# EUROPEAN PATENT APPLICATION

(11) **EP 4 555 931 A1**
(43) Date of publication of application: **21.05.2025**
(21) Application number: 24208485.3
(22) Date of filing: 23.10.2024
(51) Int. Cl.: A61B 5/339, A61B 5/346, A61B 5/00

(54) **METHODS AND SYSTEM FOR ELECTROCARDIOGRAPH (ECG) RHYTHM PRESENTATION BY A MOBILE DEVICE**

(30) Priority: 14.11.2023 US 202318509129
(71) Applicant: GE Precision Healthcare LLC, Waukesha, WI 53188 (US)
(72) Inventor: VENON, Medhi, Wauwatosa, 53226-4856 (US); YOUNG, Brian, Wauwatosa, 53226-4856 (US); OGBONNA, Benjamin, Wauwatosa, 53226-4856 (US)
(74) Representative: Fennell, Gareth Charles

(57) **Abstract**

Methods and systems are provided for organizing and displaying lead rhythm graphs of an electrocardiogram (ECG) on a mobile device. In an example, a method for a mobile device comprises receiving ECG data including a set of lead rhythm graphs, identifying an abnormality in one or more lead rhythm graphs of the set of lead rhythm graphs, ordering the set of lead rhythm graphs based on the abnormality to form an ordered set of lead rhythm graphs, such that a first lead rhythm graph of the ordered set of lead rhythm graphs includes a first waveform which includes the abnormality, and outputting for display a subset of lead rhythm graphs of the ordered set of lead rhythm graphs to a display device of a mobile device, wherein the subset of lead rhythm graphs includes at least the first lead rhythm graph.

## Description

### FIELD

Embodiments of the subject matter disclosed herein relate to automatic organization and display of ECG rhythm graphs on a mobile device.

### BACKGROUND

Electrocardiograph (ECG) monitors are widely used to obtain medical (e.g., biopotential) signals containing information indicative of electrical activity associated with the heart and pulmonary system of a patient. To obtain medical signals, ECG electrodes are applied to the skin of the patient in various locations. The electrodes, after being positioned on the patient, connect to an ECG monitor by a set of ECG lead wires. The distal end of the ECG lead wire, or portion closest to the patient, may include a connector which is adapted to operably connect to the electrode to receive medical signals from the body. The proximal end of the ECG lead set is operably coupled to the ECG monitor and supplies the medical signals received from the body to the ECG monitor. Medical signals (e.g., ECG rhythm graphs) captured by ECG monitors may include twelve lead rhythm graphs, each of which are captured using one or more ECG leads. The twelve lead rhythm graphs are presented on the ECG monitor and/or a display device, such as a mobile device, communicably coupled thereto.

### SUMMARY

In one aspect, a method for organizing and displaying electrocardiograph (ECG) rhythm data includes receiving electrocardiogram (ECG) data, including a set of lead rhythm graphs, identifying an abnormality in one or more lead rhythm graphs of the set of lead rhythm graphs, ordering the set of lead rhythm graphs based on the abnormality to form an ordered set of lead rhythm graphs, such that a first lead rhythm graph of the ordered set of lead rhythm graphs includes a first waveform which includes the abnormality, and outputting for display a subset of lead rhythm graphs of the ordered set of lead rhythm graphs to a display device of a mobile device, wherein the subset of lead rhythm graphs includes at least the first lead rhythm graph.

In this way, single lead rhythm graphs of a set of lead rhythm graphs of an ECG dataset may be displayed on a display device of a mobile device, where each lead rhythm graph is displayed for sufficient visualization of a full waveform, including details of the waveform which may clinically assist in diagnosis. Additionally, by organizing the lead rhythm graphs of the set of lead rhythm graphs in response to automatically detected abnormalities in the ECG data, a time to diagnosis may be decreased as navigation among different lead rhythm graphs to manually identify abnormalities may be decreased. Further, an accuracy of diagnosis may be increased.

The above advantages, other advantages, and features of the present description will be readily apparent from the following detailed description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows a block diagram of a patient monitoring system, including a multi-lead electrocardiograph (ECG) system and a mobile device.
FIG. 2 shows an example waveform captured by the ECG system of FIG. 1.
FIG. 3 shows an illustrative example views of the waveform of FIG. 2, displayed on the mobile device of FIG. 1, where a user navigates through different views of a first section of the waveform by interacting with the mobile device.
FIGS. 4A and 4B show an illustrative example views of the waveform of FIG. 2, displayed on the mobile device of FIG. 1, where a user navigates through different views of a second section of the waveform by interacting with the mobile device.
FIGS. 5A and 5B show an illustrative example of views of the waveform of FIG. 2, displayed on the mobile device of FIG. 1, where a user navigates through different views of a third section of the waveform by interacting with the mobile device.
FIGS. 6A and 6B show an illustrative example of views of the waveform of FIG. 2, displayed on the mobile device of FIG. 1, where a user navigates through different views of a fourth section of the waveform by interacting with the mobile device.
FIG. 7 shows a flow chart for determining how to order leads based on identified abnormalities of a waveform.
FIGS. 8A and 8B show a flow chart of a method for operating the mobile device of FIG. 1 to organize and display lead rhythm graphs of an ECG.
FIG. 9 shows a flow chart of a method for identifying abnormalities of a waveform.
FIG. 10 shows a flow chart of a method for identifying an orientation of the mobile device of FIG. 1.

### DETAILED DESCRIPTION

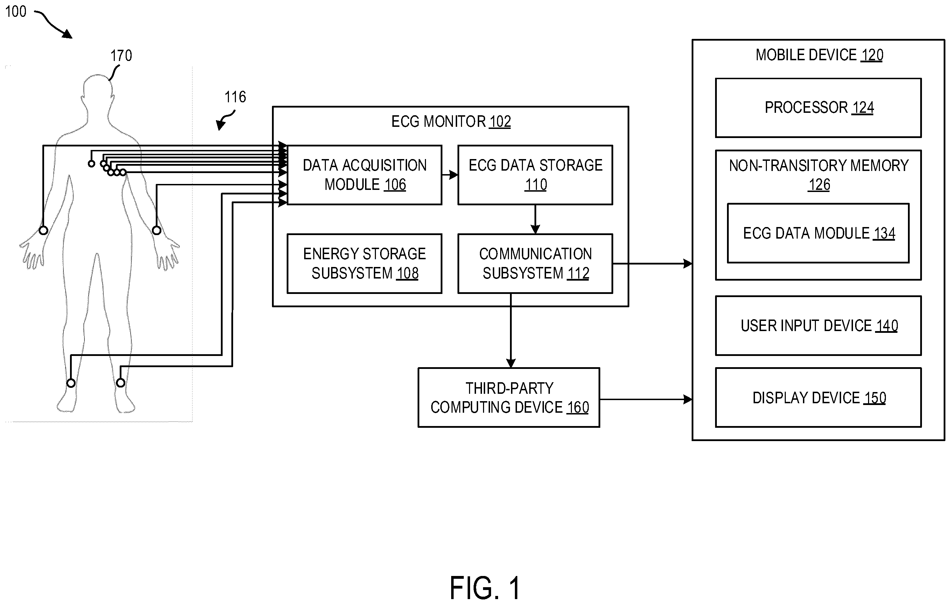

Embodiments of the present disclosure will now be described, by way of example, with reference to the FIGS. 1-10, which relate to various embodiments for providing a guided view of ECG waveforms in the form of lead rhythm graphs which fit dimensions of a mobile device and guide a user through viewing the ECG waveforms and regions of interest thereof. The mobile device may be part of and/or communicably coupled to an ECG system, and may receive a dataset including multiple lead rhythm graphs. A computing device, for example, the mobile device or a third-party computing device communicably coupled to the mobile device and the ECG system, may analyze waveforms of the multiple lead rhythm graphs and identify abnormalities of the waveforms. Information regarding identified abnormalities in the lead rhythm graphs may be sent from the third-party computing device to the mobile device, when the third-party computing device is used to analyze the waveforms. The mobile device may order the lead rhythm graphs for display on the mobile device based on relevant medical information, including the analysis of abnormalities performed by either the mobile device or the third-party computing device. For example, the mobile device may order the multiple lead rhythm graphs according to an order that arranges lead rhythm graphs that show the identified abnormality/abnormalities at a beginning of the order. A first lead rhythm graph of the ordered set of lead rhythm graphs may be output for display on a display (e.g., a touchscreen) of the mobile device. The mobile device may receive user inputs, and in response may navigate among lead rhythm graphs of the ordered set of lead rhythm graphs. A number of lead rhythm graphs output for display and an orientation of the displayed lead rhythm output graph(s) may be determined in response to a user input and/or a size and an orientation of a display of the mobile device.

Conventionally, ECG waveforms are presented in a predefined layout in which all acquired lead rhythm graphs are presented simultaneously on a single display. For example, the ECG waveforms may be presented in a Cabrera format for a twelve-lead ECG, where each of the twelve lead rhythm graphs are simultaneously output for display on a display device. A user, such as a healthcare provider, may analyze the lead rhythm graphs to identify abnormalities in the ECG waveforms. The inclusion of all twelve lead rhythm graphs on a single display device may be visually overwhelming to a user, and it may be challenging to use the mobile device, which may result in a long time of analysis in which the user analyzes each individual lead rhythm graph, identifies abnormalities in one or more of the lead rhythm graphs, and may make a diagnosis in response to identifying and comparing abnormalities in multiple of the lead rhythm graphs. Additionally, mobile devices which have a relatively small screen size (e.g., eight inches or less along a diagonal of the screen) are being used in healthcare environments to increase access to data by healthcare providers and increase freedom of mobility in healthcare (e.g., moving patients and/or providers among different rooms/areas). Providing a large amount of ECG rhythm data (e.g., twelve lead rhythm graphs) at the same time on the relatively small screen may make it challenging for a user to visualize abnormalities in ECG rhythm data. For example, a user may interact with the mobile device via a user input device of the mobile device to zoom in (e.g., enlarge) aspects of one or more lead rhythm graphs. The size of the screen of the mobile device may be too small to effectively display the ECG waveforms with all nuances of ECG waveform intervals and segments to enable patient condition diagnosis. This may result in inaccurate diagnosis and/or a long duration of analysis to make the diagnosis.

The present disclosure at least partially addresses the above described issues by providing a system which guides users through a diagnosis discovery process of the patient condition with an efficient presentation of ECG waveforms of all ECG lead rhythm graphs, based on clinical features of interest in the ECG, which are identified using an automated analysis. The system and methods described herein are designed to be implemented in a small screen mobile device, for example, a mobile device having a display device with a diagonal length of eight inches or less, and/or a foldable screen. The methods described herein address challenges of a mobile device form factor (e.g., small screen) and associated challenges with visualization of ECG waveforms by presenting ECG lead rhythm graphs in an order based on detection of abnormalities and other clinical features of interest performed by automated analysis. The systems and methods further provide a mechanism for navigating among lead rhythm graphs with minimal user input. The disclosed systems and methods provide a sequenced preview of the ECG based on the most clinically relevant features identified by a computerized ECG analysis.

Displaying selected data of interest (e.g., displaying a first lead rhythm graph having relevant medical information and, in response to receiving a user input, displaying a second lead rhythm graph having relevant medical information) allows the user to see the most relevant data without having to navigate among, select, and analyze multiple lead rhythm graphs of the set of lead rhythm graphs. The speed of a user's navigation through various views (e.g., various lead rhythm graphs) may be increased because the methods described herein for guided display (e.g., navigation) saves the user from selecting a lead rhythm graph from an array of all lead rhythm graphs of the set of lead rhythm graphs which are displayed simultaneously, manually enlarging the selected lead rhythm graph, analyzing the selected lead rhythm graph to identify abnormalities, and repeating this process to identify lead rhythm graphs of the set of lead rhythm graphs having abnormalities which may be indicative of a disease state. In conventional methods, this may include enlarging and shrinking one or more lead rhythm graphs multiple times to compare lead rhythm graphs of the set of lead rhythm graphs, and to view details of the ECG waveform of a lead rhythm graph in further detail to identify lead rhythm graphs of the set of lead rhythm graphs which include relevant medical information. In conventional displays, a size of the lead rhythm graph when all lead rhythm graphs of the set of lead rhythm graphs are displayed simultaneously on the display (e.g., in a three by four grid) may be sized to fit all lead rhythm graphs of the set of lead rhythm graphs on the display of the mobile device simultaneously, where the size may be too small to visualize details of each lead rhythm graph. Rather than stepping through selection, enlargement, and analysis of multiple lead rhythm graphs to identify relevant medical information, using the method described herein, the user may only need to view the display of the mobile device, which has a single lead rhythm graph displayed thereon which has been identified as having relevant medical information (e.g., identified abnormality). The user may interact with the mobile device (e.g., swipe or tap a touchscreen display of the mobile device) to navigate among lead rhythm graphs, where the mobile device outputs lead rhythm graphs having relevant medical information in response to receiving the user input. The claims included herein are thus directed to an improvement in the functioning of computers, particularly those with small screens (e.g., mobile devices). Additionally, conventional methods which include displaying multiple (e.g., twelve) lead rhythm graphs simultaneously may have a high demand on a processor and a memory of the computing device (e.g., the mobile device) to retrieve and display data for each of the lead rhythm graphs simultaneously. By comparison, displaying a single lead rhythm graph at a time may reduce demand on the processor and the memory, as data for the single lead rhythm graph is retrieved, rather than demand for retrieving multiple lead rhythm graphs.

The disclosed ordering and display of the lead rhythm graphs provides a specific manner of displaying a limited set of information to the user, rather than using conventional user interface methods to simultaneously display all lead rhythm graphs of the set of lead rhythm graphs. These methods recite a specific improvement over prior systems, resulting in a user interface for mobile devices that is easier to navigate and for a user to intuitively understand. The guided view of ECG waveforms disclosed herein may be advantageous because it avoids a user having to enlarge and/or shrink sections of multiple lead rhythm graphs multiple times to find desired data, thereby preventing sorting through and analyzing a large amount of data while searching for desired data, which may be slow, complex, and difficult to learn.

The disclosed invention increases the efficiency of using the mobile device by ordering the set of lead rhythm graphs based on relevant medical information which may or may not be included in each lead rhythm graph of the set of lead rhythm graphs, and outputting for display a lead rhythm graph having relevant medical information. For example, the guided view of ECG waveforms may increase the efficiency of using the computing device by bringing together and presenting the medical information most relevant to the user (via ordering of the set of lead rhythm graphs), which may allow the user to view the most relevant medical information of the set of lead rhythm graphs without accessing and analyzing each lead rhythm graph of the set of lead rhythm graphs. The speed of a user's navigation through the set of lead rhythm graphs may be increased because the disclosed guided view of ECG waveforms saves the user from enlarging and analyzing each lead rhythm graph individually to identify abnormalities in the ECG waveform. Additionally, a size of ECG data presented on the small form factor (e.g., small screen) while preserving details of the ECG data may be maximized while also minimizing effects of noise on ECG readability.

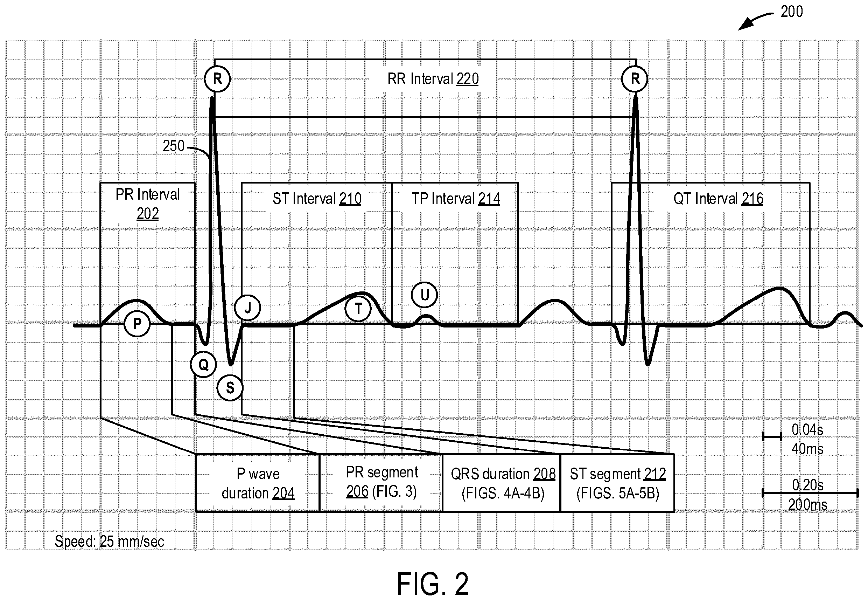

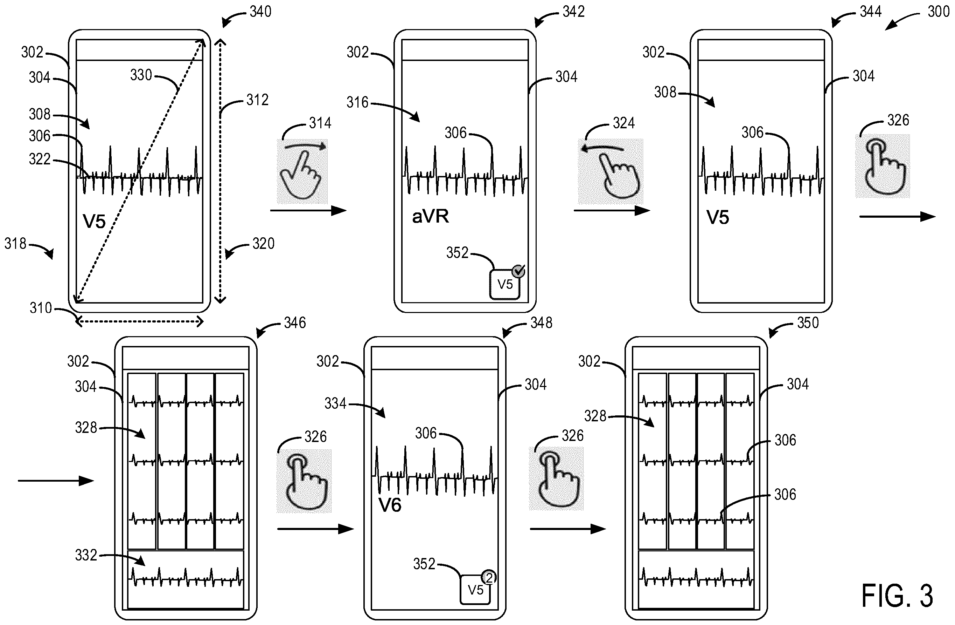

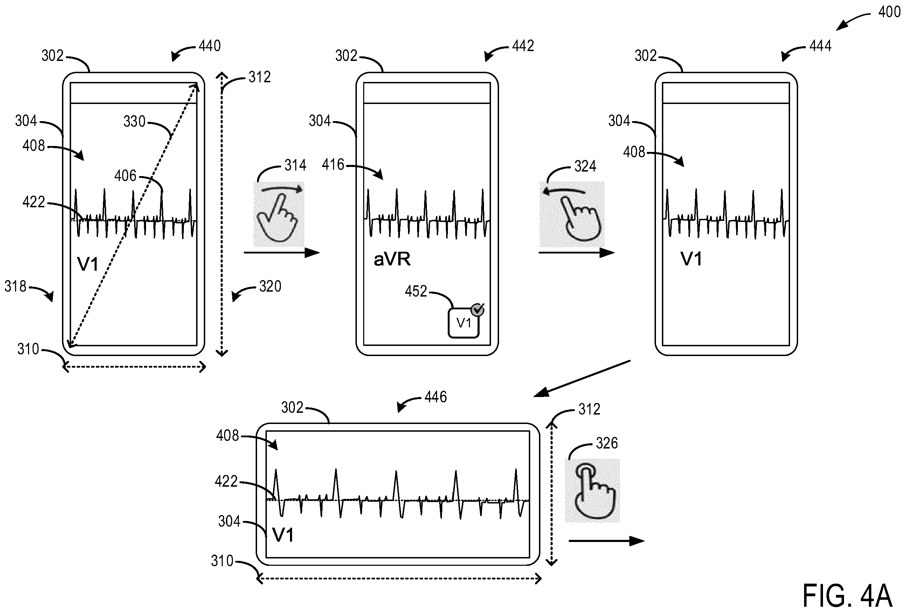

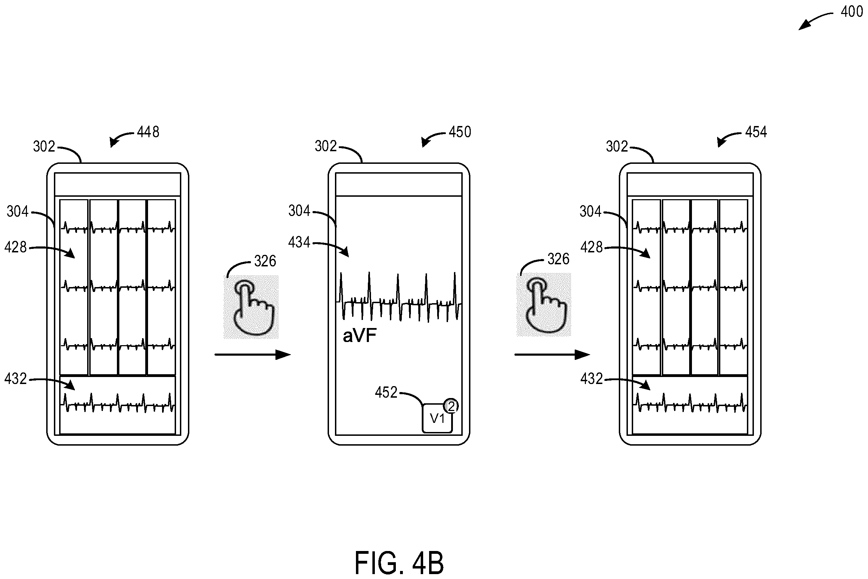

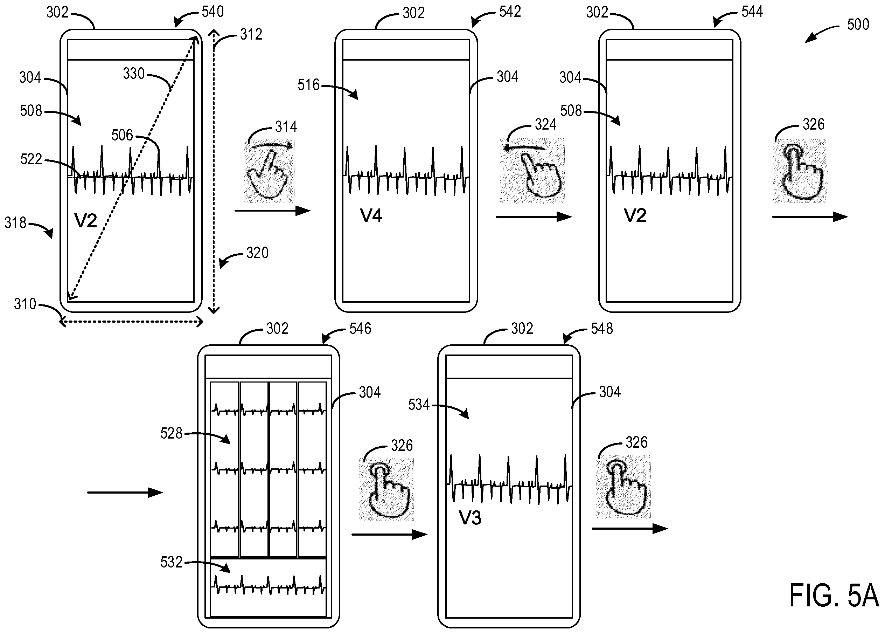

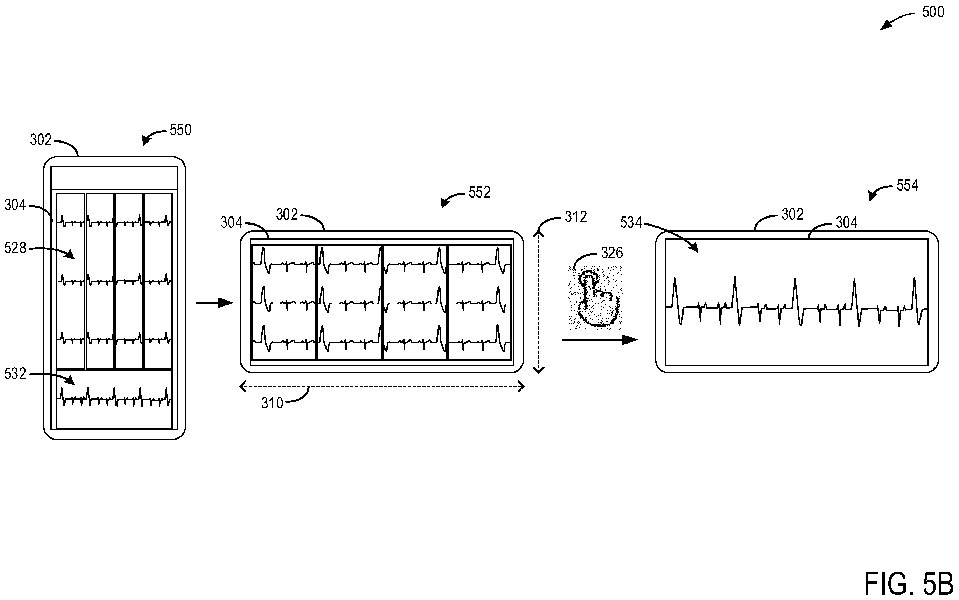

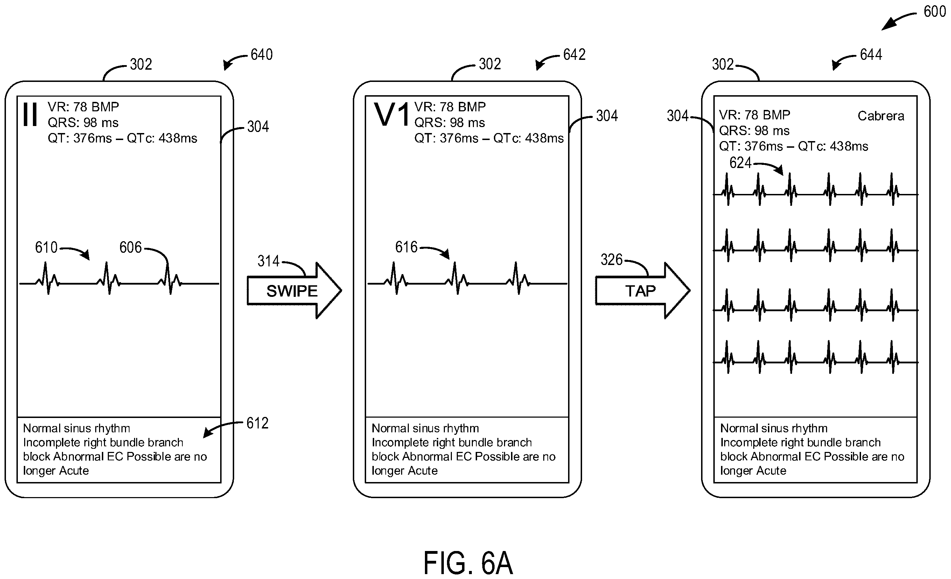

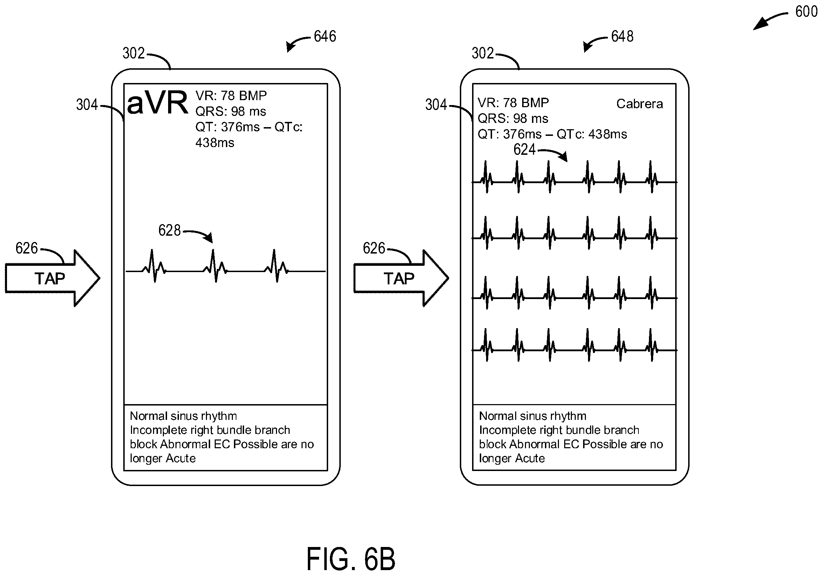

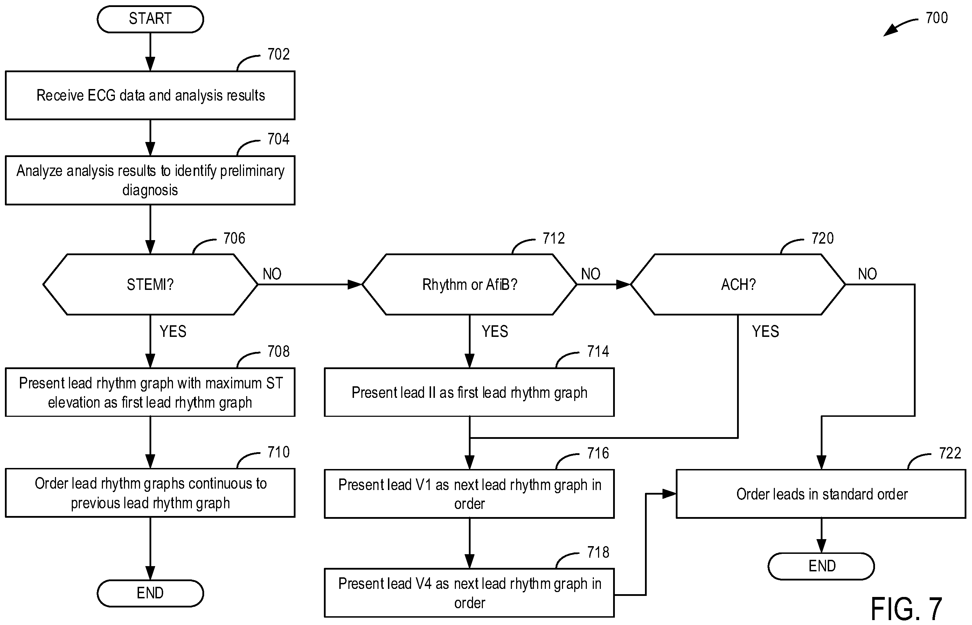

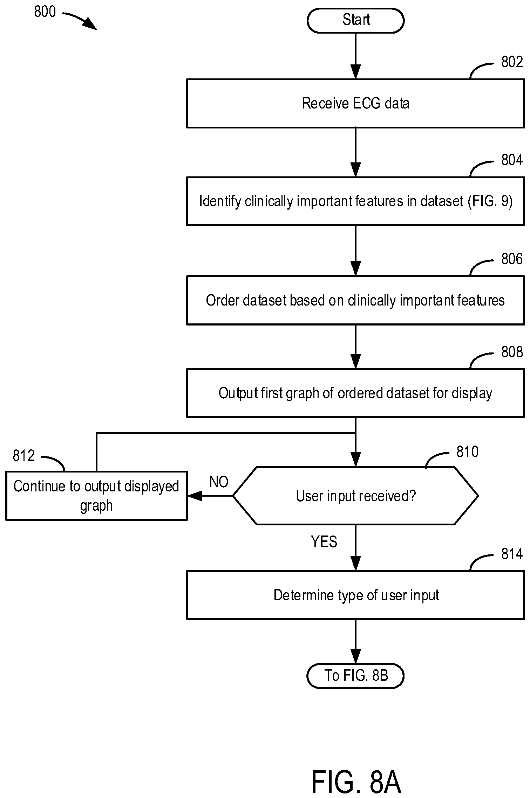

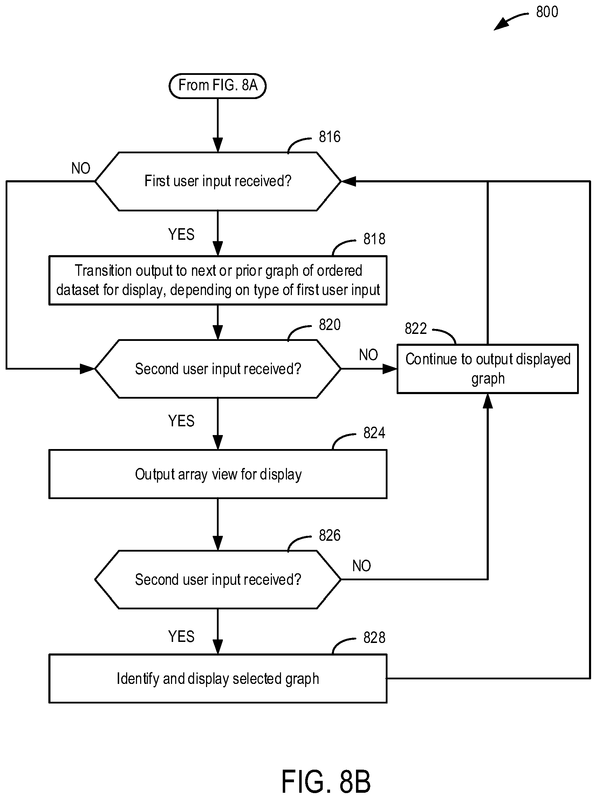

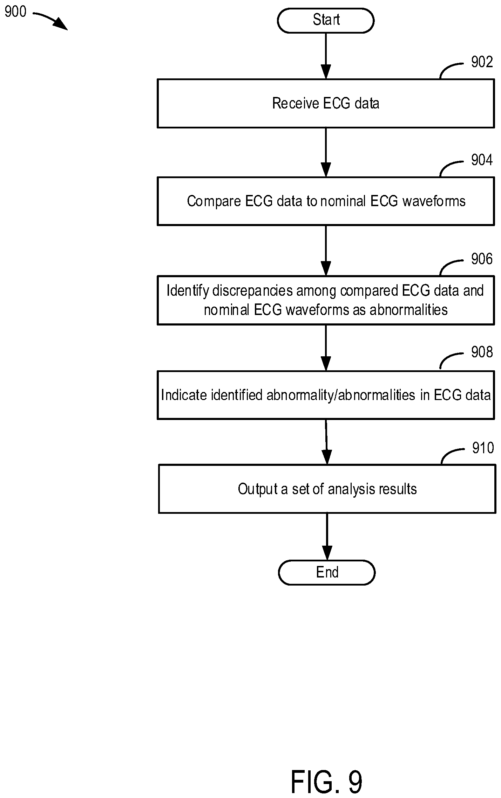

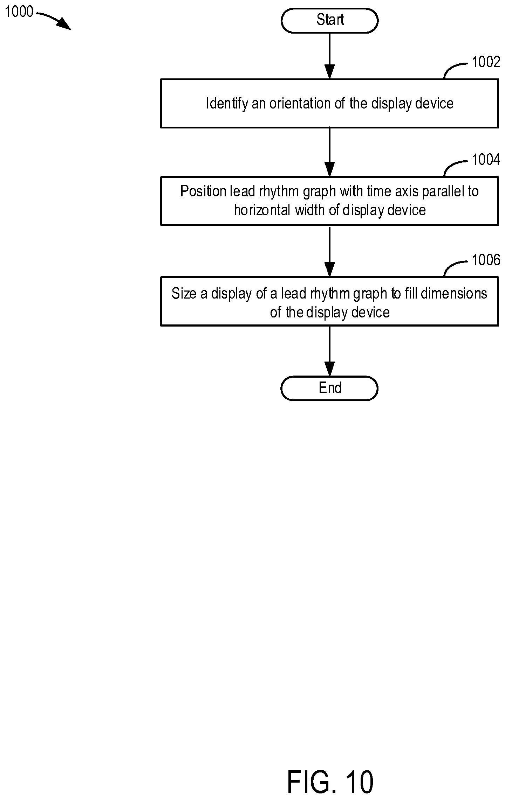

FIG. 1 shows a patient monitoring system that may be used to acquire electrocardiograph (ECG) waveform data. The ECG waveform data acquired using the patient monitoring system may include a plurality (e.g., twelve) of waveforms, each corresponding to a lead (e.g., two electrodes) of the patient monitoring system. The patient monitoring system of FIG. 1 includes a mobile device that may be used to visualize the ECG waveform data, and may be communicably coupled to a third-party computing device configured to analyze ECG waveform data to identify abnormalities in one or more ECG waveforms. An example ECG waveform which may be acquired using the patient monitoring system of FIG. 1 is shown in FIG. 2. The ECG waveform of FIG. 2 is divided into different segments and durations, each of which may be used to assist in patient diagnosis. The methods described herein include automatically ordering lead rhythm graphs (e.g., which illustrate ECG waveform data) based on detected abnormalities in the waveforms shown in a respective lead rhythm graph. For example, FIG. 3 shows an illustrative example of waveforms, specifically PR segments of the waveforms, that may be displayed on the mobile device in response to a user interacting with the mobile device to navigate through the waveforms. FIGS. 4A-6B show additional illustrative example views of different sections of the waveforms which may be shown on the display device in response to user input to the mobile device. For example, FIGS. 4A and 4B show example views of a QRS duration of each waveform, FIGS. 5A and 5B show example views of ST segments of the waveforms, and FIGS. 6A and 6B show sections of the ECG waveforms which include identified rhythm abnormalities. An order in which the lead rhythm graphs are organized and displayed, as described with respect to FIGS. 3A-6B, may be determined in response to analysis of the ECG waveforms performed by the mobile device or a third-party computing device, as described with respect to FIG. 9. Analysis of the ECG waveforms to detect abnormalities may result in preliminary diagnosis of the waveform, based on the abnormalities. FIG. 7 shows a flow chart of a method for determining how to order lead rhythm graphs based on identified abnormalities of the waveforms. FIGS. 8A and 8B show a flow chart of a method for organizing and displaying lead rhythm graphs of ECG waveform data. In some embodiments, the mobile device may have a rectangular display, and an orientation of one or more displayed lead rhythm graphs may be determined based on an orientation of the mobile device, as described with respect to FIG. 10.

Turning now to the figures, FIG. 1 shows an embodiment of a patient monitoring system 100. The patient monitoring system 100 comprises an ECG monitor 102 and a mobile computing device, referred to herein as a mobile device 120, communicably coupled thereto. The ECG monitor 102 is configured to measure and store a recording of the electrical activity of the heart of a patient 170 and comprises a plurality of electrodes 116. The ECG data acquired by the ECG monitor 102 may be transferred to the mobile device 120 for further processing before being evaluated by a healthcare professional, such as a cardiologist. The mobile device 120 is configured to receive ECG data from the ECG monitor 102, organize the ECG data in accordance with identified abnormalities in the ECG data, and output organized ECG data for display in accordance with an orientation of the mobile device and user inputs. Additionally, in some embodiments, the ECG monitor 102 may include or may be communicably coupled to a third-party computing device 160, which may be further communicably coupled to the mobile device 120 and may be configured to receive and perform analysis on ECG waveform data to identify abnormalities in the ECG waveforms, as further described herein. Further detail regarding methods for analysis, organization, and display of the ECG data is described herein with respect to FIGS. 2-10. The healthcare professional may evaluate the ECG data that is acquired by the ECG monitor 102 and output for display by the mobile device 120 for signs of an arrhythmia or another cardiac disorder.

The ECG data recorded by the ECG monitor 102 comprises time series data, wherein an electric potential difference (voltage) between two or more electrodes 116 in electrical contact with the skin of the patient 170 is recorded as a function of time. Although FIG. 1 includes the ECG monitor 102, it may be appreciated that other devices that record heart activity through time may be used, such as an inertial sensor that records periodic movement, for example. The ECG monitor may further include a controller to adjust ECG acquisition based on user input. In the embodiment shown in FIG. 1, the plurality of electrodes 116 include ten electrodes which are attached to the patient 170 via adhesive pads and/or electrically conductive gel. Thus, in the present example, the plurality of electrodes 116 are configured to measure a ten lead ECG, wherein the electric potential is measured along distinct axes passing through the heart of the patient 170. From these measured ten leads, more leads may be calculated. For example, from the measured limb leads, augmented limb leads may be calculated. Thus, this system may provide more leads than what it measures (e.g. twelve total leads). However, it may be appreciated that the ECG monitor 102 may comprise more or fewer than ten electrodes. For example, the ECG monitor 102 may measure six leads, or fifteen leads. Similarly, the placement of the electrodes 116 on the patient 170 may differ without departing from the scope of this disclosure.

When configured as a twelve lead ECG system, the patient monitoring system 100 includes three groups of leads: standard limb leads (e.g., bipolar leads), augmented limb leads (e.g., unipolar leads), and precordial/chest leads (e.g., unipolar leads). The standard limb leads include: lead I, which records electrical activity between the right arm and the left arm; lead II, which records electrical activity between the right arm and the left leg; and lead III, which records electrical activity between the left arm and the left leg. The augmented limb leads include: aVR, which record electrical activity from a virtual reference point in the center of the heart towards the right arm; aVL, which records electrical activity from a virtual reference point in the center of the heart towards the left arm; and aVF, which records electrical activity from a virtual reference point in the center of the heart towards the left leg. The precordial/chest leads include: V1, which is placed at a fourth intercostal space (e.g., of the ribs) at a right sternal border (e.g., of the sternum); V2, which is placed at the fourth intercostal space; V3, which is placed between V2 and V4; V4, which is placed at a fifth intercostal space in a midclavicular line; V5, which is placed horizontally at the same level as V4 in an anterior axillary line; and V6, which is placed horizontally at the same level as V5 in a midaxillary line. The precordial/chest leads each record electrical activity at a respective position.

The plurality of electrodes 116 may be electrically coupled to a data acquisition module 106 of the ECG monitor 102. The data acquisition module 106 is configured to measure electric potential differences between two or more of the plurality of electrodes 116, or between an electrode and a terminal as a function of time, and record the measurement in an ECG data storage 110. In some embodiments, the data acquisition module 106 may be configured to receive analog electrical signals from the plurality of electrodes 116, amplify and/or filter the analog signals, and convert the analog signals to digital signals before storing the digital signals as a function of time in the ECG data storage 110. In another embodiment, the data acquisition module 106 may convert the analog electrical signals from the plurality of electrodes 116 to a digital signal and may amplify and/or filter the digital signal before storing the digital signal as a function of time in the ECG data storage 110. The measurement of electric potential difference may be a differential between two electrodes (two potentials) or combination of potentials in case when Wilson's central terminal (WCT) potential is used as a reference when measuring a chest lead (chest-electrode potential minus WCT-potential). Often WCT-potential is not provided in analog electronics, but the chest lead is measured by referencing to RA-electrode potential (chest-electrode potential minus RA-electrode potential). In this case the WCT-referenced chest lead is calculated afterwards.

The data acquisition module 106 is communicably coupled with the ECG data storage 110 and may write ECG data acquired from the patient 170 to the ECG data storage 110. The ECG data storage 110 may comprise non-transitory memory, wherein the ECG data acquired by the data acquisition module 106 may be stored. The ECG data stored in ECG data storage 110 may comprise time series data, wherein an amplitude of the electric potential difference between two or more of the plurality of electrodes 116 or between an electrode and a terminal is recorded at regular intervals in time. For example, each recorded electric potential difference may be time stamped with the time of acquisition, thereby creating time series data. A storage capacity of the ECG data storage 110 may be selected such that an expected number of beats from one or more ECG monitor recordings may be stored thereon. In some embodiments, the ECG data storage 110 may comprise a removable component, enabling a user to physically remove the ECG data storage 110 from the ECG monitor 102. In some embodiments, the ECG data storage 110 may comprise a memory card, a flash drive, or a removable hard drive. In some embodiments, the ECG data storage 110 may be integrated into the ECG monitor 102 and may include a solid state drive (SSD), hard disk drive (HDD).

The ECG monitor 102 further comprises an energy storage subsystem 108, wherein electrical energy may be stored, enabling the ECG monitor 102 to operate while attached to a patient for hours or days without plugging the ECG monitor into an outlet. In some embodiments, the energy storage subsystem 108 comprises a rechargeable battery.

In some embodiments, a communication subsystem 112 may selectively communicably couple the ECG monitor 102, the mobile device 120, and/or the third-party computing device 160. In one embodiment, the communication subsystem 112 may comprise a wireless or wired connection configured to transfer ECG data from the ECG data storage 110 of the ECG monitor 102 to the mobile device 120 and/or to the third-party computing device 160. In some embodiments, the communication subsystem 112 may enable the ECG monitor 102, the mobile device 120, and/or the third-party computing device 160 to be in substantially continuous communication via a wireless network, enabling the mobile device 120 to receive substantially real-time ECG data from the ECG monitor 102 and/or the third-party computing device 160. As used herein, the term "real-time" refers to a process executed without intentional delay. The communication subsystem 112 may include wired and/or wireless communication devices compatible with one or more different communication protocols. As non-limiting examples, the communication subsystem 112 may be configured to transfer ECG data from the ECG data storage 110 to the mobile device 120 and/or the third-party computing device 160 via a wireless network, a wireless local area network, Wi-Fi Direct (e.g., peer to peer) a wired local area network, a wireless wide area network, a wired network, and so on. In some embodiments, the communication subsystem 112 may allow the ECG monitor 102 to send and/or receive data to and/or from other devices via a network, such as the public Internet. For example, the communication subsystem 112 may communicatively couple the ECG monitor 102 with the mobile device 120 and/or the third-party computing device 160 via a network, such as the public Internet.

ECG data acquired by the ECG monitor 102 may be transferred to the mobile device 120 for long term storage, processing (e.g., signal filtering, normalization, noise suppression, etc.), display, and analysis. In one embodiment, the mobile device 120 may comprise a processor 124 configured to execute machine readable instructions stored in a non-transitory memory 126. The processor 124 may be single core or multi-core, and the programs executed thereon may be configured for parallel or distributed processing. In some embodiments, the processor 124 may optionally include individual components that are distributed throughout two or more devices, which may be remotely located and/or configured for coordinated processing. In some embodiments, one or more aspects of the processor 124 may be virtualized and executed by remotely-accessible networked computing devices configured in a cloud computing configuration. In some embodiments, the non-transitory memory 126 may include components disposed at two or more devices, which may be remotely located and/or configured for coordinated processing. In some embodiments, one or more aspects of the non-transitory memory 126 may include remotely-accessible networked storage devices configured in a cloud computing configuration.

The non-transitory memory 126 further includes an ECG data module 134, which may include a data storage module for storing ECG monitor data collected from one or more patients. In some embodiments, the ECG data module 134 may receive ECG data from ECG monitor 102 and may store the ECG data received therefrom. In some embodiments, the mobile device 120 may receive ECG data and vital sign data from a plurality of data sources, including one or more network devices. Data stored within the ECG data module 134 may be organized according to one or more known organizational schemes or configured into one or more known data structures. In some embodiments, the ECG data may be stored in the ECG data module 134 by indexing the data according to patient, acquisition time, originating monitor ID, and so forth.

The mobile device 120 further includes a user input device 140 and a display device 150. The user input device 140 may comprise one or more of a touchscreen, a keyboard, a mouse, a trackpad, a motion sensing camera, or other device configured to enable a user to enter, interact with, and/or manipulate, data within the mobile device 120. Based on the user input, the mobile device 120 may output for display one or more rhythm lead graphs, and optionally, a rhythm summary, of ECG monitor data stored in the ECG data module 134.

The display device 150 may include one or more display devices utilizing any type of display technology, such as a monitor, touchscreen, holographic, and/or projector. In some embodiments, the display device 150 may comprise the touchscreen of the user input device 140. The display device 150 may be combined with the processor 124, the non-transitory memory 126, and/or the user input device 140 in a shared enclosure or may be a peripheral device. The display device 150 may be a relatively small screen, for example, may have a diagonal length of eight inches or less. Further detail regarding the size of the display device 150 and a role of the size of the display device 150 in displaying lead rhythm graphs of ECG monitor data is further described herein with respect to FIGS. 3-8B.

It may be understood that the patient monitoring system 100 shown in FIG. 1 is one exemplary embodiment, and other patient monitoring systems having similar components may also be possible. For example, another appropriate patient monitoring system may include more, fewer, or different components. In one example, the ECG system may be a portable or a wearable device where the ECG monitor, and one or more components of the mobile device may be included within a terminal of the ECG system. The terminal may be a portable device that may be hand held or attached to the patient via a fastener (such as a belt).

Turning to FIG. 2, an example lead rhythm graph 200 is shown, which includes an ECG waveform 250. The lead rhythm graph 200 may be generated from data captured by the data acquisition module 106 of the ECG monitor 102 of FIG. 1 using the processor 124 of the mobile device 120, as further described herein. In some embodiments, the lead rhythm graph 200 may be generated by another computing device, such as a computing device of the ECG monitor 102 or the third-party computing device 160, and sent to the mobile device 120 via a wired or wireless connection. The lead rhythm graph 200 is annotated to assist in visualizing different regions of interest in the ECG waveform 250, which may assist in diagnosis of heart conditions, as is further described with respect to FIGS. 3-5. The ECG waveform 250 is captured at a speed of 25mm/sec, and the lead rhythm graph 200 has a scale in which a small box is 0.04s (e.g., 40ms), and a large box is 0.20s (e.g., 200ms). The ECG waveform 250 includes two heart cycles (e.g., two heart beats), as further described herein.

A P wave (P) represents electrical activity of atria depolarization, which leads to atrial contraction and pumping of blood into ventricles. A QRS complex represents depolarization of the ventricles, and includes a series of waves and deflections (e.g., a Q deflection (Q), a R wave (R), and a S deflection (S)). The QRS complex indicates initiation of ventricular contraction, which results in pumping of blood to the lungs and the rest of the body. A PR interval 202 extends from a beginning of the P wave to a beginning of the QRS complex, and represents the time it takes for the electrical impulse to travel from the atria to the ventricles. The PR interval 202 includes a P wave duration 204 and a PR segment 206. As further described with respect to FIG. 3, irregularities in the PR segment 206 may indicate the presence of an atrioventricular delay, which may be caused by an atrioventricular block. The QRS complex has a QRS duration 208 which includes morphology of the QRS complex and, as further described with respect to FIGS. 4A and 4B, may be used to detect bundle branch blocks in the heart, among other abnormalities. A J point (J) represents a junction between termination of the QRS complex and the beginning of a ST interval 210, as further described herein. The J point marks an end of ventricular depolarization (e.g., contraction) and a beginning of ventricular repolarization (e.g., relaxation).

A T wave (T) represents repolarization of the ventricles and occurs as the heart prepares for the next cycle of depolarization. A ST segment 212 of the ST interval 210 follows the QRS complex and represents a period between ventricular depolarization (e.g., at the S deflection) and repolarization (e.g., at the beginning of the T wave). The ST segment 212 is at a baseline electric potential in normal heartbeats. As further described with respect to FIGS. 5A-5B, deviation from the baseline may indicate myocardial ischemia (e.g., inadequate blood flow to the heart). A TP interval 214 extends from the end of the T wave to the beginning of a next P wave. During the TP interval 214, the ECG is typically a flat line, representing a period between an end of ventricular repolarization and a beginning of atrial depolarization. In some waveforms, a U wave (U) may be present during the TP interval 214. A QT interval 216 is measured from the beginning of the QRS complex to the end of the T wave and represents a total time it takes for the ventricles to depolarize and repolarize. Extended length of the QT interval 216 may be associated with an increased potential of arrhythmias. An RR interval 220 is a period between two consecutive ventricular depolarizations (e.g., heartbeats) and may be used to calculate a heart rate (e.g., number of heart beats per minute).

Different ECG data, such as different sections of the ECG waveform 250 as shown by the example lead rhythm graph 200 may be used to assist in diagnosis of different heart conditions and abnormalities. For example, a twelve lead ECG system having ten electrodes (e.g., the patient monitoring system 100 of FIG. 1) may capture ECG data across different cardiac axes, or regions of the heart. As described with respect to FIG. 1, the twelve lead ECG system includes three groups of leads: standard limb leads (e.g., bipolar leads), augmented limb leads (e.g., unipolar leads), and precordial/chest leads (e.g., unipolar leads). Each lead provides a different view of the heart's electrical activity. For example, a lead rhythm graph of each of the respective twelve leads may differ from other lead rhythm graphs of the other leads. This may show abnormalities in specific regions and/or along specific axes of the heart, which may assist in diagnosis of heart abnormalities specific to one or more regions of the heart. Standards exist in healthcare diagnostic practices to consult different groupings of leads to assist in diagnosing different heart abnormalities. For example, grouping leads by region of the body may include lead I, aVL, V5, and V6 in a first group of lateral leads. A second group of septal leads may include V1 and V2. A third group of inferior leads may include lead II, lead III, and aVF. A fourth group of anterior leads may include V3 and V4. The aVR lead may be independently used and/or used in addition to one or more leads of the first through fourth groups. In some embodiments, different combinations of lead groupings may be used to analyze heart abnormalities.

As described herein, a method is desired which provides a guided view of ECG data which fits within dimensions of a small display device, such as a screen of a mobile device which has a diagonal length of eight inches or less. Described herein is a method for providing a sequential preview of ECG data based on clinically relevant features which are identified by a computerized ECG analysis. The computerized ECG analysis may be performed by a computing device, such as a computing device of the ECG monitor 102 or the third-party computing device 160 of FIG. 1, and/or a computing device communicably coupled to the ECG monitor 102. Results of the computerized ECG analysis may be directly or indirectly sent (e.g., via the ECG monitor 102) to a mobile device which is configured to organize and display lead rhythm graphs based on results of the computerized ECG analysis, such as the mobile device 120 of FIG. 1. In some embodiments, computerized ECG analysis may be performed by the mobile device itself, following receipt of ECG waveform data by the mobile device. As further described with respect to FIG. 9, the computerized ECG analysis may compare ECG waveform data to nominal ECG waveform data (e.g., not having abnormalities or other indicators of disease states) for relevant subject characteristics (e.g., age, height, weight, sex, and so on), and identify discrepancies in the ECG waveforms. In some embodiments, the analysis may assign a preliminary diagnosis to the ECG waveform data based on the identified abnormality/abnormalities. For example, the algorithm may identify one or more of an irregular heart rate, widening and/or a decrease in amplitude of the QRS complex, inversion of the T wave, elevated amplitude of the ST interval, and so on. When performed by a computing device other than the mobile device (e.g., the mobile device 120), following analysis, results of the analysis may be sent to the mobile device. Based on the identified abnormality, the mobile device may identify a potential group of lead rhythm graphs which show relevant medical information (e.g., characteristics in the waveforms) which may assist in diagnosis. The mobile device may order the set of lead rhythm graphs according to a desired order of data presentation associated with the identified abnormality to form an ordered set of lead rhythm graphs. For example, further detail regarding an identified abnormality in the ST segment of the ST interval may be provided in the V2, V4, and V3 leads. The mobile device may output a first lead rhythm graph of the ordered set of lead rhythm graphs for display on the display device. Outputting the first lead rhythm graph may include identifying dimensions of the display, as well as an orientation of the display, and orienting and sizing the first lead rhythm graph accordingly. Additionally, the mobile device may receive user input for navigating among lead rhythm graphs. For example, a user may input predefined user inputs, such as different gestures, which may result in the mobile device halting display of the first lead rhythm graph and outputting for display a second lead rhythm graph of the ordered list of lead rhythm graphs. Further details and examples of the methods are described herein with respect to FIGS. 3-10.

Turning to FIG. 3, a first example illustration 300 is shown of navigation among views of ECG waveforms displayed on a mobile device 302, where a user navigates through different views by interacting with the mobile device 302. The mobile device 302 is an example of a computing device, such as the mobile device 120 of FIG. 1, that includes a display device 304, a user interface, and a processor, among other features as described with respect to the mobile device 120 of FIG. 1. The mobile device 302 is configured to receive ECG data (which may include results of an ECG waveform analysis, as described with respect to FIG. 9), order lead rhythm graphs of the ECG data in an order that is based on one or more identified abnormalities in the ECG data, and output a lead rhythm graph for display on the display device 150 according to the order. In some embodiments, the mobile device 302 may receive ECG waveform data and may itself perform the ECG waveform analysis described with respect to FIG. 9 to identify abnormalities in the ECG waveforms. Additionally, the display device 304 may be configured as a touchscreen, and thus operates as both a display device and a user interface of the mobile device 302.

The illustration 300 shows navigation among views of lead rhythm graphs including different waveforms of the ECG waveform data. For example, an ECG waveform 306 may be an example of the waveform 250 of FIG. 2. The ECG waveform 306 shown on different display views of the mobile device 302 is provided for example purposes, and may not include specific morphology indicative of heart abnormalities. It is to be understood that, in practical examples, morphology of the ECG waveform 306 may be different for different lead rhythm graphs (e.g., each lead rhythm graph may show a different ECG waveform). In the example of FIG. 3, an abnormality may be detected (e.g., by the ECG waveform analysis performed by the mobile device 302, the ECG monitor, or a third-party computing device) in the PR segment of the ECG data (e.g., the PR segment 206 of FIG. 2). Each display view of FIG. 3 may show a PR segment of the ECG waveforms (e.g., the PR segment 206 of FIG. 2) captured by a different lead of a twelve-lead ECG monitor (e.g., the ECG monitor 102 of FIG. 1). The ECG waveform 306 may therefore have morphology which is different among lead rhythm graphs. For example, morphology of the PR segment as captured by a V5 lead may include a depression of the PR segment, and morphology of the PR segment as captured by a V1 lead may not include the depression of the PR segment. An abnormality in the PR segment may be indicative of different disease states, based on the type of abnormality. For example, depression of the PR segment may indicate pericarditis or atrial infarction. As the PR segment represents the time it takes for electrical impulses to travel from the atria to the ventricles, it may be helpful for diagnosis to analyze a center of the heart, as well as a lower region of the heart (e.g., the ventricles). Thus, a desired order of data presentation identified and implemented by the mobile device for the lead rhythm graphs may include V5, followed by aVR. The ordered set of lead rhythm graphs in the example of FIG. 3 includes V5, followed by aVR, and may further include additional ordering of the lead rhythm graphs of the remaining twelve leads, or may include the remaining lead rhythm graphs in a standard order.

Prior to outputting a lead rhythm graph of the ordered set of lead rhythm graphs, the mobile device 302 may identify a first orientation of the display device 304. The display device 304 of the mobile device 302 has a rectangular shape with a horizontal width 310 which is perpendicular to a direction of gravity, and a vertical length 312 which is parallel to the direction of gravity. The mobile device 302 may generally be positioned in one of two orientations. In a first (e.g., vertical) orientation, the horizontal width 310 of the display device 304 is less than the vertical length 312 of the display device 304. In a second (e.g., horizontal) orientation, the mobile device 302 may be rotated 90 degrees relative to the first orientation, such that the horizontal width 310 of the display device 304 is greater than the vertical length 312, as further described herein with respect to FIGS. 4A-6B. A diagonal length 330 of the display device 304 may be used to describe a relative size of the display device 304 and thus of the mobile device 302. For example, the diagonal length 330 may be eight inches or less, which may define the mobile device 302 as having a relatively small display (e.g., screen, compared to a display device of a tablet, a computer monitor, and so on). The display device 304 of the mobile device 302 may be, for example, a touch screen or other device which may simultaneously operate as a display device and as a user input device. The mobile device may identify an orientation and a display size of the mobile device 302, as further described with respect to FIG. 10. For example, as shown in FIG. 3, the mobile device 302 is in a first orientation (e.g., a vertical orientation). The mobile device may orient a lead rhythm graph in response to the determined orientation of the mobile device 302. When the display device 304 is in the first orientation, a first lead rhythm graph is output for display with a time axis 322 parallel to the horizontal width 310 of the display device. The time axis 322 is shown in the first view 340 as a dashed line, and may or may not be visible in the first lead rhythm graph 308 and/or subsequent views of FIG. 3.

Following ordering of the set of lead rhythm graphs to form an ordered set of lead rhythm graphs, a first lead rhythm graph 308 of the ordered set of lead rhythm graphs may be output for display in a first view 340. The first lead rhythm graph 308 may be displayed on the display device 304, and no other lead rhythm graphs of the ordered set of lead rhythm graphs may be displayed in the first view 340. In the example of FIG. 3, the first lead rhythm graph 308 is the V5 lead rhythm graph. The display device 304 may show a ten second interval of the first lead rhythm graph 308.As further described herein, the first lead rhythm graph 308 may be positioned as the first lead rhythm graph of the ordered set of lead rhythm graphs based on characteristics of the ECG waveform shown in the first lead rhythm graph 308. For example, the ECG waveform of the first lead rhythm graph 308 may include a depression of the PR segment, which may indicate a disease state of the heart. By outputting the first lead rhythm graph 308 in the first view 340, relevant medical information may be presented to the user (e.g., displayed on the display device 304), which may enable the user to avoid having to navigate through multiple lead rhythm graphs of the set of lead rhythm graphs to identify one or more lead rhythm graphs having medically relevant information. The first lead rhythm graph 308 may be displayed in response to a user request to view ECG waveform data on the mobile device 302. The first lead rhythm graph 308 may thus be in a launched state (e.g., displayed) while other lead rhythm graphs of the ordered set of lead rhythm graphs are in an unlaunched state (e.g., not displayed).

In response to receiving a first user input 314, the mobile device 302 may transition the first lead rhythm graph 308 from the launched state to the unlaunched state, and transition a second lead rhythm graph 316 from the unlaunched state to the launched state. A second view 342 thus includes the second lead rhythm graph 316 output for display and no other lead rhythm graphs of the ordered set of lead rhythm graphs being output for display. The second lead rhythm graph 316 is the aVR lead rhythm graph, which directly follows (e.g., without an intervening step between) the first lead rhythm graph 308 in the ordered set of lead rhythm graphs. The first user input 314 is a swipe from a left side 318 of the display device 304 to a right side 320 of the display device 304. The first user input 314 requests a transition to a next lead rhythm graph in the ordered set of lead rhythm graphs. In some embodiments, the second view 342 includes a view counter 352 which indicates that a lead rhythm graph other than the lead rhythm graph shown in the second view 342 has been previously viewed. For example, the view counter 352 indicates that the V5 lead rhythm graph (e.g., the first lead rhythm graph 308) has been viewed by including a check mark in the view counter 352.

In response to receiving a second user input 324, the mobile device 302 may transition the second lead rhythm graph 316 from the launched state to the unlaunched state, and may transition the first lead rhythm graph 308 from the unlaunched state to the launched state. A third view 344 thus includes the first lead rhythm graph 308 output for display and no other lead rhythm graphs of the ordered set of lead rhythm graphs being output for display. The second user input 324 is a swipe from the right side 320 of the display device 304 to the left side of the display device 304. In another example where the first user input 314 is a swipe from the right side 320 of the display device 304 to the left side 318 of the display device 304, the second user input 324 may be a swipe from the left side 318 of the display device to the right side 320 of the display device. The second user input 324 requests a transition to a previous lead rhythm graph of the ordered set of lead rhythm graphs.

In response to receiving a third user input 326 when a single lead rhythm graph of the ordered set of lead rhythm graphs is in the launched state (e.g., is displayed), the mobile device 302 may launch two or more of the ordered lead rhythm graphs which are in the unlaunched state (e.g., not displayed). A fourth view 346 thus includes an array 328 of each of the twelve lead rhythm graphs output for display in response to the third user input 326. The third user input 326 may be a tap anywhere on the display device 304 (e.g., which also operates as the user input device). A configuration of the array 328 may be automatically chosen by the mobile device 302 in response to the size of the display device 304. For example, the ordered set of lead rhythm graphs may be displayed in an order from left to right, where the first lead rhythm graph 308 is shown in a top left corner and a last lead rhythm graph is shown in a bottom right corner of the display device 304. In the example shown in FIG. 3, the array 328 includes three rows of four lead rhythm graphs, where each lead rhythm graph is of an equal size. In some embodiments, the array 328 further includes an additional display 332 of the lead rhythm graph displayed immediately prior to receipt of the third user input 326 (e.g., the first lead rhythm graph 308) or a rhythm summary, as further described with respect to FIGS. 6A-6B. The display device 304 may have a diagonal length 330 of eight inches or less, for example. In embodiments where the diagonal length is less than eight inches (e.g., 7.5in, 6in, and so on), the array 328 may have a different configuration. For example, the array may be configured in four rows of three lead rhythm graphs, and may exclude the additional display 332.

In response to receiving the third user input 326 (e.g., the tap) when the array 328 of ordered lead rhythm graphs is displayed, the mobile device 302 may transition all of the lead rhythm graphs of the ordered set of lead rhythm graphs to the unlaunched state (e.g., not displayed) except for a selected lead rhythm graph 334 which is selected using the third user input 326. For example, a user may tap on any one of the lead rhythm graphs displayed in the array 328, and the mobile device may maintain the selected lead rhythm graph 334 in the launched state (e.g., displayed). A fifth view 348 therefore includes a single lead rhythm graph. In the example of FIG. 3, the selected lead rhythm graph 334 is the V6 lead rhythm graph. When displayed in the array 328, the V6 lead rhythm graph may be sized the same as the other lead rhythm graphs of the array 328. When the other lead rhythm graphs of the array 328 are transitioned to the unlaunched state (e.g., no longer displayed), the size of the selected lead rhythm graph 334 may be increased to fill the size of the display device 304. In some embodiments, the view counter 352 may be included in the fifth view 348. For example, the view counter 352 indicates that the V5 lead rhythm graph (e.g., the first lead rhythm graph 308) has been viewed twice.

The mobile device 302 may receive either of the first user input 314 and the second user input 324 at any time when a single rhythm lead graph is output for display (e.g., in the launched state) and transition to output a directly following or directly preceding lead rhythm graph in the ordered set of lead rhythm graphs, respectively, when applicable. For example, if the mobile device 302 receives the second user input 324 when the first lead rhythm graph 308 is output for display, the mobile device 302 may not navigate to another lead rhythm graph. In another example, the mobile device 302 may navigate to a last lead rhythm graph of the ordered set of lead rhythm graphs upon receiving the second user input 324 when the first lead rhythm graph 308 is in the launched state. Additionally, the mobile device 302 may receive the third user input 326 at any time and, in response, display an array of two or more of the ordered set of lead rhythm graphs when a single lead rhythm graph is in the launched state, or display a selected lead rhythm graph from a displayed array of two or more of the ordered set of lead rhythm graphs. For example, the mobile device 302 may receive the third user input 326 when the fifth view 348 is output for display, and may transition to a sixth view 350 which includes the array 328 of multiple lead rhythm graphs.

Turning to FIGS. 4A and 4B, a second example illustration 400 is shown of navigation among different views of an ECG waveform displayed on the mobile device 302 of FIG. 3, where a user navigates through different views by interacting with the mobile device 302. For example, each display view of FIGS. 4A and 4B may show a QRS duration of an ECG waveform (e.g., the QRS duration 208 of FIG. 2) that has been captured by a different lead of a twelve-lead ECG monitor (e.g., the ECG monitor 102 of FIG. 1). A waveform 406 shown on the display device 304 is provided for example purposes, and may not include specific morphology indicative of heart abnormalities. It is to be understood that, in practical examples, the waveform 406 may be different for different lead rhythm graphs. In some lead rhythm graphs, the ECG waveform 406 may have morphology which is different from other lead rhythm graphs. In the example of FIGS. 4A and 4B, an abnormality may be detected in the QRS duration of the ECG data (e.g., the QRS duration 208 of FIG. 2). For example, morphology of the QRS duration as captured by a V1 lead may include a prolonged QRS duration, and morphology of the QRS duration as captured by a V5 lead may not include the prolonged QRS duration. An abnormality in the QRS duration may be indicative of different disease states. For example, a prolonged QRS duration may indicate delayed ventricular electrical activation, such as a left bundle-branch block. It may be helpful for diagnosis to analyze a center of the heart from both a left side and a right side. Thus, a desired order of data presentation for the lead rhythm graphs may include V1, followed by aVR. The ordered set of lead rhythm graphs in the example of FIGS. 4A and 4B includes V1, followed by aVR, and may further include additional ordering of the lead rhythm graphs of the remaining twelve leads, or may include the remaining lead rhythm graphs in a standard order.

Prior to outputting the first lead rhythm graph 408 for display, the mobile device may identify an orientation and a display size of the mobile device 302. For example, as shown in FIGS. 4A and 4B, the mobile device 302 may be in a first orientation, where the horizontal width 310 of the display device 304 is less than the vertical length 312 of the display device, or may be in a second orientation, where the horizontal width 310 of the display device 304 is greater than the vertical length 312 of the display device 304. When the display device 304 is in the first orientation, a lead rhythm graph is output for display with a time axis 422 parallel to the horizontal width 310 of the display device.

Following ordering of the set of lead rhythm graphs to form an ordered set of lead rhythm graphs, a first lead rhythm graph 408 of an ordered set of lead rhythm graphs may be output for display to the display device 304 in a first view 440. In the example of FIGS. 4A and 4B, the first lead rhythm graph 408 is the V1 lead rhythm graph. The first lead rhythm graph 408 may be output for display, and no other lead rhythm graphs of the ordered set of lead rhythm graphs may be output for display. The display device 304 may show a ten second interval of the first lead rhythm graph 408. As further described herein, the first lead rhythm graph 408 may be positioned as the first lead rhythm graph of the ordered set of lead rhythm graphs based on characteristics of the ECG waveform shown in the first lead rhythm graph 408. For example, the ECG waveform of the first lead rhythm graph 408 may include a prolonged QRS duration, which may indicate a disease state of the heart. By outputting the first lead rhythm graph 408 in the first view 440, relevant medical information may be presented to the user (e.g., displayed on the display device 304), which may enable the user to avoid having to navigate through multiple lead rhythm graphs of the set of lead rhythm graphs to identify one or more lead rhythm graphs having medically relevant information. The first lead rhythm graph 408 may be displayed in response to a user request to view ECG waveform data on the mobile device 302. The first lead rhythm graph 408 may thus be in a launched state (e.g., displayed) while other lead rhythm graphs of the ordered set of lead rhythm graphs are in an unlaunched state (e.g., not displayed).

In response to receiving the first user input 314, the mobile device 302 may transition the first lead rhythm graph 408 from the launched state to the unlaunched state, and transition a second lead rhythm graph 416 from the unlaunched state to the launched state. A second view 442 thus includes the second lead rhythm graph 416 output for display and no other lead rhythm graphs of the ordered set of lead rhythm graphs being output for display. The second lead rhythm graph 416 is the aVR lead rhythm graph, which directly follows (e.g., without an intervening lead rhythm graph between) the first lead rhythm graph 408 in the ordered set of lead rhythm graphs. The first user input 314 is a swipe from a left side 318 of the display device 304 to a right side 320 of the display device 304. For example, the display device 304 may be a touch screen which also operates as a user input device. As the mobile device 302 is still in the first orientation and the display device 304 size is unchanged, the second lead rhythm graph 416 is output for display with the time axis 422 parallel to the horizontal width 310. The first user input 314 requests a transition to a next lead rhythm graph in the ordered set of lead rhythm graphs. In some embodiments, the second view 442 includes a view counter 452 which indicates that a lead rhythm graph other than the lead rhythm graph shown in the second view 442 has been previously viewed. For example, the view counter 452 indicates that the V1 lead rhythm graph (e.g., the first lead rhythm graph 408) has been viewed by including a check mark in the view counter 452.

In response to receiving the second user input 324, the mobile device 302 may transition the second lead rhythm graph 416 from the launched state to the unlaunched state, and may transition the first lead rhythm graph 408 from the unlaunched state to the launched state. A third view 444 thus includes the first lead rhythm graph 408 output for display and no other lead rhythm graphs of the ordered set of lead rhythm graphs being output for display. The second user input 324 is a swipe from the right side 320 of the display device 304 to the left side of the display device 304. In another example where the first user input is a swipe from the right side 320 of the display device 304 to the left side 318 of the display device 304, the second user input 324 may be a swipe from the left side 318 of the display device to the right side 320 of the display device.

An orientation of the mobile device 302 may be changed at any time, and a display of one or more lead rhythm graphs on the display device 304 may automatically adjust accordingly. For example, when displaying the first lead rhythm graph 408, the mobile device 302 may change from the first orientation to the second orientation, where in the second orientation the horizontal width 310 is greater than the vertical length 312. An orientation of the first lead rhythm graph 408 may accordingly be changed, so that the time axis 422 of the first lead rhythm graph 408 remains parallel to the horizontal width 310. For example, the mobile device 302 may transition from the third view 444 to a fourth view 446, where the fourth view 446 includes the same lead rhythm graph as is displayed in the third view 444 (e.g., the first lead rhythm graph 408). When in the second orientation, the display device 304 may show a same or a different portion of the first lead rhythm graph 408. For example, the same ten second interval of the first lead rhythm graph 408 may be shown when the mobile device 302 is in the first orientation or the second orientation. When shown in the second orientation, the waveform 406 may be expanded, relative to when shown in the first orientation, and may retain relative scaling to preserve accuracy of the data. In other embodiments, an interval which is greater than or less than ten seconds may be shown when the mobile device 302 is in the second orientation, if the data is available (e.g., included in the ECG data received by the mobile device 302).

In response to receiving the third user input 326 when a single lead rhythm graph (e.g., the first lead rhythm graph 408) of the ordered set of lead rhythm graphs is in the launched state and the mobile device 302 is in the second orientation (e.g., the fourth view 446), the mobile device 302 may transition from the single lead rhythm graph to an array 428 of multiple lead rhythm graphs of the ordered set of lead rhythm graphs, as shown in a fifth view 448 of FIG. 4B. The third user input 326 may be a tap anywhere on the display device 304 (e.g., which also operates as the user input device). For example, if the first lead rhythm graph 408 is displayed on the display device 304, the mobile device 302 is rotated from the first orientation to the second orientation, and the mobile device 302 receives the third user input 326, the mobile device 302 may decrease a size of the first lead rhythm graph 408 and output for display the array 428 of multiple lead rhythm graphs. The fifth view 448 may further include display of the first lead rhythm graph. The time axis of each lead rhythm graph of the ordered set of lead rhythm graphs is parallel to the horizontal width 310. A configuration of the array 428 may be automatically chosen by the mobile device 302 in response to the size of the display device 304. For example, the ordered set of lead rhythm graphs may be displayed in an order from left to right, where the first lead rhythm graph 408 is shown in a top left corner and a last lead rhythm graph is shown in a bottom right corner of the display device 304. In the example shown in FIGS. 4A-4B, the array 428 includes three rows of four lead rhythm graphs, where each lead rhythm graph is of an equal size. In some embodiments, the array 428 further includes an additional display 432 of the lead rhythm graph displayed immediately prior to receipt of the third user input 326 (e.g., the first lead rhythm graph 408) or a rhythm summary, as further described with respect to FIGS. 6A-6B. The display device 304 may have the diagonal length 330 of eight inches or less, for example. In embodiments where the diagonal length is less than eight inches (e.g., 7.5in, 6in, and so on), the array 428 may have a different configuration. For example, the array may be configured in four rows of three lead rhythm graphs, and may exclude the additional display 432.

In response to receiving the third user input 326 (e.g., the tap) when the array 428 of ordered lead rhythm graphs is displayed, the mobile device 302 may transition all of the lead rhythm graphs of the ordered set of lead rhythm graphs to the unlaunched state except for a selected lead rhythm graph 434 which is selected using the third user input 326. For example, a user may tap on any one of the lead rhythm graphs displayed in the array 428, and the computing device may maintain the selected lead rhythm graph 434 in the launched state. A sixth view 450 thus includes a single lead rhythm graph. In the example of FIGS. 4A-4B, the selected lead rhythm graph 434 is the aVF lead rhythm graph. When displayed in the array 428, the aVF lead rhythm graph may be sized the same as the other lead rhythm graphs of the array 428. When the other lead rhythm graphs of the array are transitioned to the unlaunched state, the size of the selected lead rhythm graph 434 may be increased to fill the size of the display device 304. For example, the display device 304 may show a ten second interval of the selected lead rhythm graph 434. In some embodiments, the view counter 452 may be included in the sixth view 450. For example, the view counter 452 indicates that the V1 lead rhythm graph (e.g., the first lead rhythm graph 408) has been viewed twice.

The mobile device 302 may receive either of the first user input 314 and the second user input 324 at any time when a single rhythm lead graph is output for display (e.g., in the launched state) and transition to output a directly following or directly preceding lead rhythm graph in the ordered set of lead rhythm graphs, respectively, when applicable. For example, if the mobile device 302 receives the second user input 324 when the first lead rhythm graph 408 is output for display, the mobile device 302 may not navigate to another lead rhythm graph. In another example, the mobile device 302 may navigate to a last lead rhythm graph of the ordered set of lead rhythm graphs upon receiving the second user input 324 when the first lead rhythm graph 408 is in the launched state. Additionally, the mobile device 302 may receive the third user input 326 at any time and, in response, display an array of two or more of the ordered set of lead rhythm graphs when a single lead rhythm graph is in the launched state, or display a selected lead rhythm graph from a displayed array of two or more of the ordered set of lead rhythm graphs. For example, the mobile device 302 may receive the third user input 326 when the sixth view 450 is output for display, and may transition to a seventh view 454 which includes the array 328 of multiple lead rhythm graphs.

Turning to FIGS. 5A and 5B, a third example illustration 500 of navigation among lead rhythm graphs is shown. FIGS. 5A and 5B include a mobile device 302 with a display device 304, which may be an example of the mobile device 302 of FIGS. 3-4B, and thus includes the components described therein (e.g., processor, memory, and so on). A waveform 506 shown on the display device 304 is provided for example purposes, and may not include specific morphology indicative of heart abnormalities. It is to be understood that, in practical examples, the waveform 506 may be different for different lead rhythm graphs.

Upon receiving ECG data (e.g., a set of lead rhythm graphs), the processor of the mobile device 302 may execute an algorithm to detect abnormalities in the ECG data. In other examples, a device which sends the ECG data to the mobile device 302 may perform the algorithm analysis. For example, the device may be the ECG monitor 102 of FIG. 1, or another medical device communicably coupled to the ECG monitor 102 and the mobile device 302. In the example of FIGS. 5A and 5B, an abnormality may be detected in the ST segment of the ECG data (e.g., the ST segment 212 of FIG. 2). An abnormality in the ST segment may be indicative of different disease states. For example, an elevated waveform within the ST segment may indicate myocarditis, pericarditis, or other challenges with ventricular depolarization and repolarization. It may be helpful for diagnosis to analyze ventricular regions of the heart. Thus, a desired order of data presentation for the lead rhythm graphs may include V2, followed by V4. The ordered set of lead rhythm graphs in the example of FIGS. 5A and 5B includes V2, followed by V4, and may further include additional ordering of the lead rhythm graphs of the remaining twelve leads, or may include the remaining lead rhythm graphs in a standard order.

Prior to outputting the first lead rhythm graph 508 for display, the computing device may identify an orientation and a display size of the mobile device 302. For example, as shown in FIGS. 5A and 5B, the mobile device 302 may be in the first orientation. When the display device 304 is in the first orientation, the first lead rhythm graph 508 is output for display with a time axis 522 of the first lead rhythm graph 508 parallel to the horizontal width 310 of the display device 304. Further detail regarding identifying an orientation of the display device is described with respect to FIG. 10.

The computing device of the mobile device 302 may output a first lead rhythm graph 508 of an ordered set of lead rhythm graphs to the display device 304 in a first view 540. In the example of FIGS. 5A and 5B, the first lead rhythm graph 508 is the V2 lead rhythm graph. The display device 304 may show a ten second interval of the first lead rhythm graph 508. As further described herein, the first lead rhythm graph 508 may be positioned as the first lead rhythm graph of the ordered set of lead rhythm graphs based on characteristics of the ECG waveform shown in the first lead rhythm graph 508. For example, the ECG waveform of the first lead rhythm graph 508 may include an elevated waveform within the ST segment, which may indicate a disease state of the heart. By outputting the first lead rhythm graph 508 in the first view 540, relevant medical information may be presented to the user (e.g., displayed on the display device 304), which may enable the user to avoid having to navigate through multiple lead rhythm graphs of the set of lead rhythm graphs to identify one or more lead rhythm graphs having medically relevant information. The first lead rhythm graph 408 may be displayed in response to a user request to view ECG waveform data on the mobile device 302. The first lead rhythm graph 408 may thus be in a launched state (e.g., displayed) while other lead rhythm graphs of the ordered set of lead rhythm graphs are in an unlaunched state (e.g., not displayed).

In response to a first user input 314, the mobile device 302 may transition the first lead rhythm graph 508 from the launched state to the unlaunched state, and transition a second lead rhythm graph 516 from the unlaunched state to the launched state (e.g., output for display on the display device 304). Thus, a second view 542 displays the second lead rhythm graph 516 and does not display other lead rhythm graphs of the ordered set of lead rhythm graphs. The second lead rhythm graph 516 is the V4 lead rhythm graph, which directly follows the first lead rhythm graph 508 in the ordered set of lead rhythm graphs. The first user input 314 is a swipe from a left side 318 of the display device 304 to a right side 320 of the display device 304. For example, the display device 304 may be a touch screen which also operates as a user input device. As the mobile device 302 is still in the first orientation and the display device 304 size is unchanged, the second lead rhythm graph 516 is output for display with the time axis 522 parallel to the horizontal width 310.

In response to receiving a second user input 324, the mobile device may transition the second lead rhythm graph 516 from the launched state to the unlaunched state, and may transition the first lead rhythm graph 508 from the unlaunched state to the launched state. Thus, a third view 544 displays the first lead rhythm graph 508 and does not display other lead rhythm graphs of the ordered set of lead rhythm graphs. The second user input 324 is a swipe from the right side 320 of the display device 304 to the left side of the display device 304. In another example where the first user input is a swipe from the right side 320 of the display device 304 to the left side 318 of the display device 304, the second user input 324 may be a swipe from the left side 318 of the display device to the right side 320 of the display device.

In response to a third user input 326 when a single lead rhythm graph of the ordered set of lead rhythm graphs is in the launched state, the mobile device 302 may launch two or more of the ordered lead rhythm graphs which are in the unlaunched state. For example, an array 528 of each of the twelve lead rhythm graphs may be output for display in a fourth view 546. The third user input 326 may be a tap anywhere on the display device 304 (e.g., which also operates as the user input device). As the mobile device 302 is in the first orientation, the time axis of each lead rhythm graph of the ordered set of lead rhythm graphs is parallel to the horizontal width 310. A configuration of the array 528 may be automatically chosen by the mobile device 302 in response to the size of the display device 304. For example, the ordered set of lead rhythm graphs may be displayed in an order from left to right, where the first lead rhythm graph 508 is shown in a top left corner and a last lead rhythm graph is shown in a bottom right corner of the display device 304. In the example shown in FIGS. 5A and 5B, the array 528 includes three rows of four lead rhythm graphs, where each lead rhythm graph is of an equal size. In some embodiments, the array 528 further includes an additional display 532 of the lead rhythm graph displayed immediately prior to receipt of the third user input 326 (e.g., the first lead rhythm graph 508) or a rhythm summary, as further described with respect to FIGS. 6A-6B. The display device 304 may have the diagonal length 330 of eight inches or less, for example. In embodiments where the diagonal length is less than eight inches (e.g., 7.5in, 6in, and so on), the array 528 may have a different configuration. For example, the array may be configured in four rows of three lead rhythm graphs, and may exclude the additional display 532.

In response to receiving the third user input 326 (e.g., the tap) when the array 528 of ordered lead rhythm graphs is displayed (e.g., the fourth view 546), the mobile device 302 may transition all of the lead rhythm graphs of the ordered set of lead rhythm graphs to the unlaunched state except for a selected lead rhythm graph 534 which is selected using the third user input 326 (e.g., a fifth view 548). For example, a user may tap on any one of the lead rhythm graphs displayed in the array 528, and the mobile device may maintain the selected lead rhythm graph 534 in the launched state. In the example of FIGS. 5A and 5B, the selected lead rhythm graph 534 is the V3 lead rhythm graph. When displayed in the array 528, the V3 lead rhythm graph may be sized the same as the other lead rhythm graphs of the array 528. When the other lead rhythm graphs of the array are transitioned to the unlaunched state, the size of the selected lead rhythm graph 534 may be increased to fill the size of the display device 304. For example, the display device 304 may show a ten second interval of the selected lead rhythm graph 534. Thus, the fifth view 548 displays the selected lead rhythm graph 534 and does not display other lead rhythm graphs of the set of lead rhythm graphs. The mobile device 302 may receive the third user input 326 when a single lead rhythm graph is displayed (e.g., the fifth view 548), and may transition to display the array 528 (e.g., a sixth view 550).

An orientation of the mobile device 302 may be changed at any time, and a display of one or more lead rhythm graphs on the display device 304 may automatically adjust accordingly. For example, when displaying the array 528 of the ordered set of lead rhythm graphs (e.g., the sixth view 550), the mobile device may change from the first orientation to the second orientation, where in the second orientation the horizontal width 310 is greater than the vertical length 312. In a seventh view 552, an orientation of the array 528 may accordingly be changed, so that the time axis 522 of each lead rhythm graph remains along the horizontal width 310. When in the second orientation, the display device 304 may show a same or a different portion of each lead rhythm graph, and may show a same or a different number of lead rhythm graphs of the ordered set of lead rhythm graphs. For example, an additional display 532 of a lead rhythm graph and/or a rhythm summary may be excluded from the display when the mobile device 302 is in the second orientation. When shown in the second orientation, the waveform 506 of each lead rhythm graph may be expanded, relative to when shown in the first orientation, and may retain relative scaling to preserve accuracy of the data. In other embodiments, an interval which is greater than or less than ten seconds may be shown when the mobile device 302 is in the second orientation, if the data is available (e.g., included in the ECG data received by the mobile device 302).

In response to receiving the third user input 326 (e.g., the tap) when the array 528 of ordered lead rhythm graphs is displayed (e.g., the seventh view 552), the mobile device 302 may transition all of the lead rhythm graphs of the ordered set of lead rhythm graphs to the unlaunched state except for a selected lead rhythm graph 534 which is selected using the third user input 326. For example, a user may tap on any one of the lead rhythm graphs displayed in the array 528, and the mobile device may maintain the selected lead rhythm graph 534 in the launched state in an eighth view 554. When displayed in the array 528, the selected lead rhythm graph may be sized the same as the other lead rhythm graphs of the array 528. When the other lead rhythm graphs of the array are transitioned to the unlaunched state, the size of the selected lead rhythm graph 534 may be increased to fill the size of the display device 304. For example, the display device 304 may show a ten second interval of the selected lead rhythm graph 534.

The mobile device 302 may receive either of the first user input 314 and the second user input 324 at any time when a single rhythm lead graph is output for display (e.g., in the launched state) and transition to output a directly following or directly preceding lead rhythm graph in the ordered set of lead rhythm graphs, respectively, when applicable. For example, if the mobile device 302 receives the second user input 324 when the first lead rhythm graph 508 is output for display, the mobile device 302 may not navigate to another lead rhythm graph. In another example, the mobile device 302 may navigate to a last lead rhythm graph of the ordered set of lead rhythm graphs upon receiving the second user input 324 when the first lead rhythm graph 508 is in the launched state. Further, a user may input the first user input 314, the second user input 324, or the third user input 326 multiple times in succession to navigate among lead rhythm graphs. Additionally, the mobile device 302 may receive the third user input 326 at any time and, in response, display an array of two or more of the ordered set of lead rhythm graphs when a single lead rhythm graph is in the launched state, or display a selected lead rhythm graph from a displayed array of two or more of the ordered set of lead rhythm graphs.

FIGS. 6A and 6B show a fourth example illustration 600 of navigation among lead rhythm graphs. FIGS. 6A and 6B include a mobile device 302 of FIG. 3 with a display device 304, which may be an example of the mobile device 120 of FIG. 1, and thus includes the components described therein (e.g., processor, memory, and so on). A waveform 606 shown on the display device 304 is provided for example purposes, and may not include specific morphology indicative of heart abnormalities. It is to be understood that, in practical examples, the waveform 606 may be different for different lead rhythm graphs. In the example of FIGS. 6A and 6B, the analysis to identify abnormalities in the ECG data may be performed by a device other than the mobile device, such as the ECG monitor 102 of FIG. 1, or a medical processing device communicably coupled to an ECG monitor and to the mobile device. Analysis of the ECG data may include executing an algorithm which compares ECG data to known clinically relevant diagnostic markers to identify abnormalities in the ECG data, as further described with respect to FIG. 7. The algorithm may output a rhythm summary, and in some embodiments may include one or more markers in the ECG data to indicate abnormalities, compared to ECG data from patients not presenting with heart conditions. The rhythm summary may include an initial diagnosis based on identified abnormalities, as further described herein.

The mobile device 302 receives the ECG data, which may include markups or other indicators for identified abnormalities in the ECG data, and a rhythm summary. The mobile device may analyze the ECG data and identify an order in which to organize the set of lead rhythm graphs included in the ECG data to present first in the order one or more lead rhythm graphs which include clinically relevant characteristics (e.g., abnormalities) which may assist in guiding diagnoses. In the example of FIGS. 6A and 6B, the mobile device may identify an abnormal rhythm indicator and, as a result, order the set of lead rhythm graphs such that lead II is first in the order.

A first view 640 of the mobile device 302 on the display device 304 thereof may include the waveform 606 of a first lead rhythm graph 610 (e.g., lead II), as well as additional patient information. For example, a limited list of information regarding the ECG data is included in the first view 640, as well as subsequent displays including one or more lead rhythm graphs, as further described herein. The limited list of information may include both quantitative and qualitative data. In the example of FIGS. 6A and 6B, quantitative data includes ventricular repolarization (VR), a QRS duration, and a QT duration, which may be identified by the mobile device 302. In other embodiments, the quantitative data may include additional or alternative metrics. The first view 640 further includes a rhythm summary 612. As described above, the rhythm summary 612 may include an initial diagnosis based on identified abnormalities, as well as summarizing statements regarding clinically relevant elements of the ECG data. The rhythm summary 612 may be edited and updated in response to a user input. For example, the user input device of the mobile device 302 may include a microphone, and a user may verbally dictate notes to be included in the rhythm summary 612. In further examples, the user may type notes to be included in the rhythm summary 612 via a keyboard coupled to the mobile device 302 and/or via a touch screen (e.g., the display device 304) of the mobile device 302.

As described with respect to FIGS. 3-5B, the computing device of the mobile device 302 may receive one or more of a first user input (e.g., a swipe in a first direction indicating desired navigation to a directly subsequent lead rhythm graph), a second user input (e.g., a swipe in a second direction, opposite the first direction, indicating desired navigation to a directly previous lead rhythm graph), and a third user input (e.g., a tap, indicating desired navigation to an array of the ordered set of lead rhythm graphs). In response to receiving a first user input 314, the mobile device transitions the first lead rhythm graph 610 from a launched state to an unlaunched state, and transitions a second lead rhythm graph 616 from the unlaunched state to the launched state. The second lead rhythm graph 616 is of the V1 lead in the example of FIGS. 6A and 6B, and immediately follows lead II in the ordered set of lead rhythm graphs. As described above, the limited list of data is also displayed with the second lead rhythm graph 616. Thus, a second view 642 of the mobile device 302 includes the second lead rhythm graph 616, the rhythm summary 612, and quantitative data of the ECG data.

In response to a third user input 326 when a single lead rhythm graph of the ordered set of lead rhythm graphs is in the launched state, the mobile device 302 may launch two or more of the ordered lead rhythm graphs which are in the unlaunched state. For example, a third view 644 of the mobile device 302 includes an array 624 of eight of the twelve lead rhythm graphs. The third user input 326 may be a tap anywhere on the display device 304 (e.g., which also operates as the user input device). A configuration of the array 624 may be automatically chosen by the mobile device 302 in response to the size of the display device 304. For example, as described with respect to FIGS. 3-5B, in some embodiments the array may include all twelve lead rhythm graphs of the ordered set of lead rhythm graphs. In the example of FIGS. 6A and 6B, a size of the display device 304 of the mobile device 302 may be the same as or less than the size of the display device of the mobile device of FIGS. 3-5B. To compensate for a smaller display device size and/or to adhere to a desired size of each lead rhythm graph in the array 624, some but not all of the lead rhythm graphs may be included in the array 624. Further, in some embodiments, the ECG data of FIGS. 6A and 6B may be captured by an eight lead ECG monitor, which generates eight lead rhythm graphs, all of which are shown in the array 624. The displayed lead rhythm graphs may be displayed in a Cabrera configuration. The third view 644 which includes the array 624 further includes the quantitative and qualitative data included in each of the first view 640 and the second view 642.

In response to receiving the third user input 326 (e.g., the tap) when the array 624 of ordered lead rhythm graphs is displayed, the mobile device 302302 may transition all of the lead rhythm graphs of the ordered set of lead rhythm graphs to the unlaunched state except for a selected lead rhythm graph 626 which is selected using the third user input 326. For example, a user may tap on any one of the lead rhythm graphs displayed in the array 624, and the computing device may maintain the selected lead rhythm graph 628 in the launched state. In the example of FIGS. 6A and 6B, the selected lead rhythm graph 628 is the aVR lead rhythm graph. When displayed in the array 624, the aVR lead rhythm graph may be sized the same as the other lead rhythm graphs of the array 624. When the other lead rhythm graphs of the array 624 are transitioned to the unlaunched state, the size of the selected lead rhythm graph 628 may be increased to fill the size of the display device 304. For example, the display device 304 may show a ten second interval of the selected lead rhythm graph 628. A fourth view 646 of the mobile device 302 thus includes the selected lead rhythm graph 628, as well as the quantitative and qualitative data included in prior displays of the mobile device 302.

The mobile device 302 may receive either of the first user input 314 and a second user input (not shown) at any time when a single rhythm lead graph is output for display (e.g., in the launched state) and transition to output a directly following or directly preceding lead rhythm graph in the ordered set of lead rhythm graphs, respectively, when applicable. Additionally, the mobile device 302 may receive the third user input 326 at any time and, in response, display an array of two or more of the ordered set of lead rhythm graphs when a single lead rhythm graph is in the launched state, or display a selected lead rhythm graph from a displayed array of two or more of the ordered set of lead rhythm graphs (e.g., transition from the fourth view 646 to a fifth view 648). The quantitative data and qualitative data may be displayed in each display which includes a single lead rhythm graph, and in displays which include the array of the set of lead rhythm graphs. The qualitative data (e.g., the rhythm summary 612) may be edited and updated at any time, in response to user input to the mobile device 302.

FIG. 7 shows a flow chart illustrating a method 700 for determining how to order leads based on identified abnormalities of a waveform. As described with respect to FIGS. 1 and 3-6B, the mobile device 120, the ECG monitor 102, and/or the third-party computing device 160 communicably coupled to both the mobile device 120 and the ECG monitor 102 may execute an algorithm to identify abnormalities in the ECG data. For example, the algorithm may be a 12SL ECG analysis program, or another algorithm which compares ECG data to known clinically relevant diagnostic markers to identify abnormalities in the ECG data. The method 700 may be implemented by the mobile device (e.g., mobile device 120 of FIG. 1) following receipt of ECG data and analysis results. For example, method 700 may be carried out according to instructions stored in memory of the mobile device and executed by one or more processors of the mobile device. The detected abnormalities described with respect to FIG. 7 (e.g., STEMI, rhythm abnormality, AfiB, and ACH) are presented as example abnormalities that may be detected using the methods described herein, and are non-limiting examples of abnormalities which may be detected using the same logic.

At 702, method 700 includes receiving ECG data and analysis results. The analysis results may include a rhythm summary generated by the algorithm, in some embodiments, where the rhythm summary may include identified abnormalities, as well as an initial diagnosis based on identified abnormalities, as described with respect to FIG. 9. In other embodiments, the mobile device may perform algorithmic analysis of the ECG data to identify abnormalities and generate analysis results and, in some embodiments, a preliminary diagnosis based on the analysis results.

At 704, the method 700 includes analyzing the analysis results to identify a preliminary diagnosis generated by the algorithm, or to generate an initial diagnosis based on identified abnormalities. For example, the analysis results may include quantitative (e.g., data metrics) and/or qualitative (e.g., written summary) indicating which ECG waveforms of lead rhythm graphs include abnormalities.

The method 700 proceeds to step through a series of initial diagnoses to determine an order in which to order the set of lead rhythm graphs of the ECG data. FIG. 7 shows a first example order of initial diagnoses in the flow chart, however in other examples, the diagnoses may be referred to in a different order than described herein. At 706, method 700 determines whether the identified abnormalities and/or the initial diagnoses indicates a ST-elevation myocardial infarction (STEMI). If a STEMI is indicated, the method 700 proceeds to 708 to identify a lead rhythm graph having a ECG waveform with a highest (e.g., maximum) ST elevation. The lead rhythm graph with the highest ST elevation is positioned as a first lead rhythm graph of an ordered set of lead rhythm graphs. The method 700 may proceed to 710 to order lead rhythm graphs continuous to a previous lead rhythm graph. For example, lead rhythm graphs may be ordered such that a previous lead of a standardized order of lead rhythm graphs immediately follows the first lead rhythm graph. The remaining lead rhythm graphs may be subsequently ordered by decreasing ST elevation, or may be ordered based on a standardized order of lead rhythm graphs.

If it is determined at 706 that the ECG data does not indicate STEMI, the method 700 proceeds to 712 to determine if the algorithm detected any abnormalities in heart rhythm or atrial fibrillation (AfiB). If a rhythm abnormality (e.g., an abnormal rhythm indicator) or AfiB is detected, the method 700 proceeds to 714. At 714, the method 700 includes presenting lead II as the first lead rhythm graph in the ordered set of lead rhythm graphs. The method 700 continues to order additional lead rhythm graphs. At 716, the method 700 includes presenting lead V1 as a next lead rhythm graph in the order (e.g., following lead II as the first lead rhythm graph). At 718, the method 700 includes presenting lead V4 as a next lead rhythm graph in the order (e.g., following lead V1 as the second lead rhythm graph). The method 700 proceeds to 722 to order remaining lead rhythm graphs of the set of lead rhythm graphs in a standardized order of lead rhythm graphs.

If it is determined at 712 that the ECG data does not indicate a rhythm abnormality or AfiB, the method 700 proceeds to 720 determine if the algorithm detected abnormalities indicative of acetylcholine related disease states (ACH). If ACH is indicated in the analysis results, the method 700 proceeds to 716 to present the lead VI as the first lead rhythm graph in the ordered set of lead rhythm graphs. At 718, the method 700 includes presenting lead V4 as a next lead rhythm graph in the order (e.g., following lead V1 as the second lead rhythm graph). The method 700 proceeds to 722 to order remaining lead rhythm graphs of the set of lead rhythm graphs in a standardized order of lead rhythm graphs.

Turning now to FIGS. 8A and 8B, an example method 800 for organizing and presenting ECG data, such as lead rhythm graphs, on a mobile device is shown. The mobile device may be an example of the mobile device 120 of FIG. 1, and the method 800 is described with regard to the mobile device 120 of FIG. 1. Example implementations of the method 800 are described with respect to FIGS. 3-6B. It may be appreciated that the method 800 may be implemented with other systems and components without departing from the scope of the present disclosure. The method 800 may be carried out according to instructions stored in non-transitory memory of a mobile computing device, such as the mobile device 120 of FIG. 1.

At 802, ECG data is received by the mobile device. The ECG data includes a set of lead rhythm graphs of an ECG. For example, the set of lead rhythm graphs may include twelve lead rhythm graphs captured by a twelve lead ECG having ten electrodes. In other embodiments, the set of lead rhythm graphs may include less than twelve lead rhythm graphs. The ECG data may be captured by a patient monitoring system, such as the ECG monitor 102 of FIG. 1, and sent via wireless or wired communication to the mobile device. In some embodiments, the ECG data may be sent to the mobile device from a database which stores ECG data for multiple patients, such as a database of a hospital.

At 804, the method optionally includes identifying clinically relevant features in the ECG dataset. As described with respect to FIGS. 3-7, analysis of the ECG dataset is performed to identify abnormalities which may assist in diagnosis of heart conditions. In some embodiments, the analysis may further include identifying qualitative and quantitative data to assist in diagnosis, and/or may include a preliminary diagnosis (e.g., prior to medical provider review of the ECG data). The analysis to identify clinically relevant features may be performed by the mobile device and, in other embodiments, may be performed by another device directly or indirectly coupled to the mobile device, such as the ECG monitor 102 or another medical processing device coupled to a database to which the mobile device is also communicably coupled.

At 806, the ECG data is ordered based on clinically relevant features. As described herein, the order in which to order the ECG dataset may be determined in response to identified clinically relevant features. For example, for abnormalities detected in the ECG waveform related to ventricles of the heart, lead rhythm graphs which show detail of the ECG waveform across a related axis may be shown first in the order. In this way, the mobile device may generate and store an ordered list of lead rhythm graphs from the lead rhythm graphs of the ECG data.

At 808, the method includes outputting a first lead rhythm graph of the ordered set of lead rhythm graphs for display on a display device of the mobile device. At operation 808, a single lead rhythm graph (e.g., the first lead rhythm graph) is displayed. In some embodiments, display of the first lead rhythm graph may further include quantitative and qualitative data of the ECG data, including data of the first lead rhythm graph, which is in a launched state, and other lead rhythm graphs of the ordered set of lead rhythm graphs which are in an unlaunched state. In embodiments where the ECG data received by the mobile device includes rhythm summary data, or in embodiments where the mobile device generates initial diagnoses based on ECG data analysis, the rhythm summary may be output for display on the display device alongside (e.g., above, below, etc.) the first lead rhythm graph. Dimensions of the first lead rhythm graph, including a shape and a size of the lead rhythm graph, may be adjusted in accordance with a shape and a size of the display device, as described with respect to FIGS. 3-7.

At 810, the method includes determining if a user input is received. As further described herein, the user input may be one of a first type of user input, such as a swipe across the display screen indicating a desired move to a previous lead rhythm graph or a subsequent lead rhythm graph in the ordered set of lead rhythm graphs, or a tap indicating a desired move to an array of two or more of the ordered set of lead rhythm graphs. The user input may be received via a user input device of the mobile device. For example, the user input device may be integrated within the display device, such as a touch screen of the display device. If it is determined a user input is not received, method proceeds to 812 and continues to output the lead rhythm graph which is displayed (e.g., the first lead rhythm graph). If it is determined a user input is received, the method proceeds to 814 to determine a type of the user input.

At 816, the method includes determining if a first user input is received. As described with respect to the method 800, the first user input may be a swipe in either of a first direction or a second direction, where a swipe in a first direction (e.g., from a right side of the display device to a left side of the display device) may indicate desired transition to an immediate next lead rhythm graph in the ordered set of lead rhythm graphs. The swipe in the second direction (e.g., from the left side of the display device to the right side of the display device) may indicate desired transition to an immediate prior lead rhythm graph in the ordered set of lead rhythm graphs.

In response to receipt of the first user input, the method proceeds to 820 to transition output to the display device from the current lead rhythm graph to either the next or the prior lead rhythm graph, depending on the type of first user input. For example, if the first user input is determined to be a swipe in the first direction, the first lead rhythm graph in the ordered set of lead rhythm graphs may be transitioned from the launched state to the unlaunched state, and the second lead rhythm graph may be transitioned from the unlaunched state to the launched state. As further described herein, if the displayed lead rhythm graph is a graph other than the first lead rhythm graph, in response to receiving the first user input as a swipe in the second direction, the displayed lead rhythm graph may be transitioned from the launched state to the unlaunched state, and an immediately preceding lead rhythm graph in the ordered set of lead rhythm graphs may be transitioned from the unlaunched state to the launched state. In some embodiments, when a swipe in the second direction is received when the first lead rhythm graph is displayed, the display may transition from the first lead rhythm graph to a last lead rhythm graph of the ordered set of lead rhythm graphs, or the display may not change and may continue to display the first lead rhythm graph.

If the first user input is not received at 816, the method proceeds to 820 to determine if a second user input is received. Additionally, following operation 818, the method may continue to output the newly displayed lead rhythm graph until detection of a user input. At 820, the method includes determining if a second user input is received. As described with respect to the method 800, the second user input is a tap on the user input device, which is also the display device (e.g., a touch screen) of the mobile device. If the second user input is not received, the method proceeds to 822 and continues to output the displayed lead rhythm graph.

If, at 820, the second user input is received, the method proceeds to 824 to output an array view for display on the display device. As described with respect to FIGS. 3-6B, the array view may include two or more lead rhythm graphs of the ordered set of lead rhythm graphs. In some embodiments, the number of lead rhythm graphs included in the array may be determined in response to a size and an orientation of the display device. For example, if a display device is within a first size range (e.g., a diagonal length between six inches and eight inches), the array may include eight lead rhythm graphs of a set of twelve lead rhythm graphs. If the display device is within a second size range which is greater than the first size range (e.g., a diagonal length of greater than eight inches), the array may include all of the lead rhythm graphs of the set of lead rhythm graphs. Further, the array may have different configurations in different embodiments. For example, the array may present the ordered set of lead rhythm graphs in order, where the first lead rhythm graph is in a top left corner of the display device and the last lead rhythm graph is in a bottom right corner of the display device. In another embodiment, the array may be configured to order the lead rhythm graphs in a standardized configuration, such as a Cabrera array.

The method proceeds to continue monitoring whether a user input has been received via the display device. At 826, the method determines whether the second user input is received. If the second user input is received when the array of the ordered set of lead rhythm graphs is displayed on the display device, at 828, the method includes identifying a selected graph and display the selected graph. For example, when the array includes twelve leads of a set of lead rhythm graphs, a user may tap on one of the lead rhythm graphs in the array to select that lead rhythm graph. The method may then transition all of the lead rhythm graphs of the array except for the selected lead rhythm graph to the unlaunched state. In some embodiments, the method may further identify a region of interest within the selected graph, such as an abnormality identified by the algorithm as described above, and show the region of interest on the display device. If at 826 the second user input is not received, the method may return to 822 and continue to output the displayed graph. The method may return to 816 to continue monitoring for receipt of the first user input or the second user input.

Turning to FIG. 9, an example method 900 for analyzing ECG data (e.g., waveforms of lead rhythm graphs) is shown. The method 900 may be performed by a mobile computing device, such as the mobile device 120 of FIG. 1. In other embodiments, the method 900 may be performed by a computing device, other than a mobile computing device, which is communicably coupled to the mobile computing device. For example, the method 900 may be performed by the ECG monitor 102 of FIG. 1 and/or by the third-party computing device 160 of FIG. 1. It may be appreciated that the method 900 may be implemented with other systems and components without departing from the scope of the present disclosure. The method 900 is described herein with regard to the third-party computing device 160, and may be carried out according to instructions stored in a non-transitory memory of the third-party computing device 160.

At 902, the method 900 includes receiving ECG data. As described with respect to FIG. 1, the third-party computing device may be communicably coupled to the ECG monitor 102 of FIG. 1, which captures ECG data. The third-party computing device may further be communicably coupled to other imaging systems and/or to a database configured to store ECG data. The ECG data may include waveforms captured by a multi-lead (e.g., twelve-lead) ECG system, which may be stored in a data structure such as a set of lead rhythm graph, where each lead rhythm graph shows a waveform captured by the respective lead(s).

At 904, the method 900 includes comparing ECG data to nominal ECG waveforms. Comparing ECG data to nominal ECG waveforms may include identifying a set of nominal ECG waveforms to compare the ECG data to. For example, nominal ECG waveforms may be ECG waveforms which are stored in a database accessible by the computing device, where the nominal ECG waveforms have been analyzed by a medical provider and determined to not having abnormalities or other indicators of disease states present in the respective waveform. ECG data may be compared to nominal ECG waveforms captured from subjects having the same or similar subject characteristics (e.g., age, height, weight, sex, and so on). In some embodiments, comparing ECG data to nominal ECG waveforms may include comparing quantitative and qualitative characteristics of the ECG data, such as approximate lengths, durations, and/or amplitudes of segments and intervals of the ECG waveforms.

At 906, the method 900 includes identifying discrepancies among compared ECG data and nominal ECG waveforms as abnormalities. For example, a PR segment (e.g., the PR segment 206 of FIG. 2) may have an elevation within a first range in nominal ECG data waveforms. In the ECG data being analyzed, if the PR segment has an elevation which is greater than the first range, the PR segment may be identified as an abnormality in the ECG data.

At 908, the method 900 includes indicating an abnormality or abnormalities in the ECG data. In some embodiments, more than one abnormality may be identified at 906. For example, the operation 906 may compare all data of the ECG data to the nominal ECG waveforms and identify all discrepancies as abnormalities. At 908, one or more of the abnormalities may be indicated in the ECG data. For example, a lead rhythm graph of the set of lead rhythm graphs having the waveform with the identified abnormality may be tagged, flagged, or otherwise given an indicator to identify the lead rhythm graph as having relevant medical information (e.g., the abnormality).

At 910, the method 900 includes outputting a set of analysis results, where the set of analysis results includes the indicated abnormality or abnormalities in the ECG data. In some embodiments, the ECG data received at 902 may be modified such that lead rhythm graphs of the set of lead rhythm graphs which include abnormalities are tagged, as described with respect to operation 908. In this scenario, outputting the set of analysis results may include outputting a modified set of ECG data. In other embodiments, the ECG data may be unchanged by the method 900, and the set of analysis results may include a list of and/or a subset of lead rhythm graphs which include identified abnormalities. The set of analysis results may be output to one or more devices communicably coupled to the third-party computing device (or other computing device performing the method 900), such as the ECG monitor 102 or the mobile device 120 of FIG. 1, or a database configured to store ECG data, as described above. Outputting the set of analysis results which includes indicated abnormalities enables the first lead rhythm graph to be launched from an unlaunched state in response to detection of an abnormal rhythm indicator in the first lead rhythm graph by the mobile computing device, as described with respect to method 800 of FIGS. 8A and 8B.

Turning now to FIG. 10, an example method 1000 is shown for identifying an orientation of the display device of the mobile device, and positioning and sizing display of a lead rhythm graph accordingly. The method 1000 may be performed by a mobile computing device, such as the mobile device 120 of FIG. 1. It may be appreciated that the method 1000 may be implemented with other systems and components without departing from the scope of the present disclosure. The method 1000 is described herein with regard to the mobile device 120, and may be carried out according to instructions stored in a non-transitory memory of the mobile device 120. As described with respect to FIGS. 3-6B, the mobile device 302 (e.g., an example of the mobile device 120 of FIG. 1) has a rectangular shape with a horizontal width which is approximately perpendicular to a direction of gravity, and a vertical length which is approximately parallel to the direction of gravity. The mobile device may generally be positioned in one of two orientations. In a first (e.g., vertical) orientation, the horizontal width of the display device is less than the vertical length of the display device. In a second (e.g., horizontal) orientation, the mobile device may be rotated 90 degrees relative to the first orientation, such that the horizontal width of the display device is greater than the vertical length.

At 1002, the method 1000 includes identifying an orientation of the display device. This may include using a positioning device of the mobile device (e.g., a global positioning system) to identify a relative positon of the horizontal width and the vertical length, with respect to a direction of gravity. The method 1000 may determine if the display device is in the first orientation or the second orientation, as described above.

At 1004, the method 1000 includes positioning a lead rhythm graph with a time axis parallel to the horizontal width of the display device. As described with respect to FIGS. 3-6B, the lead rhythm graph (e.g., the first lead rhythm graph, the second lead rhythm graphs, lead rhythm graphs of the array, and so on) may be positioned such that the time axis is parallel to the horizontal axis of the display device when the display device is in the first orientation and in the second orientation.

At 1006, the method 1000 includes sizing a display of a lead rhythm graph to fill dimensions of the display device. In some embodiments, a same section of the lead rhythm graph may be displayed when the display device is in the first orientation and in the second orientation. For example, the lead rhythm graph may include a ten second waveform. When the display device is in both the first orientation and the second orientation, the full ten second waveform may be output for display on the display device. For example, when the display device is in the first orientation, the ten second waveform may be sized (e.g., compressed) in such a way that does not distort characteristics of the waveform (e.g., amplitudes, durations, and so on), while enabling the full ten second waveform to be shown on the display device. In another example, the ten second waveform may be sized (e.g., expanded/stretched) to fill the display device when the display device is in the second orientation, where sizing the waveform does not distort characteristics of the waveform (e.g., amplitudes, durations, and so on). In other embodiments, different sections of the lead rhythm graph may be output for display when the display device is in the first orientation compared to the second orientation. For example, when the display device is in the first orientation, the horizontal width is less than the vertical length. A first section of the lead rhythm graph (e.g., a first five second interval of the ten second waveform) may be output for display when the display device is in the first orientation. When the display device is in the second orientation (e.g., the horizontal width is greater than the vertical length), a second section of the lead rhythm graph (e.g., the ten second waveform may be output for display. Thus, the second section includes the first section.

As shown in FIGS. 3-6B and described with respect to FIGS. 7-10, the methods described herein may provide for an improved user interface for computing devices, and specifically for mobile devices which may have a relatively small display size (e.g., a diagonal length of eight inches or less). By ordering the lead rhythm graphs in such a way that lead rhythm graphs having clinically relevant characteristics (e.g., abnormalities which may indicate various heart diseases or conditions), outputting a first lead rhythm graph, and optionally outputting quantitative and qualitative ECG data summaries in addition to one or more lead rhythm graphs, the methods described herein may summarize ECG data in a way that enables an increase in a speed of a user's navigation through various views (e.g., various lead rhythm graphs). A user may navigate among views of a single lead rhythm graph and an array of two or more lead rhythm graphs, where the array of lead rhythm graph further enables selection of a lead rhythm graph presented in the array to be individually launched for further examination. As the rhythm summaries, including potential diagnoses as well as quantitative data summaries, are displayed when one or more of the lead rhythm graphs are in the unlaunched state, data regarding the whole set of lead rhythm graphs may be accessed when a single lead rhythm graph is in the launched state. This may enable the user to visualize and compare details, such as waveform abnormalities, of a single or multiple lead rhythm graphs with summarizing data of the ECG dataset. Lead rhythm graphs which are in the unlaunched state are accessible via user inputs, such as a first user input (e.g., a first swipe in a first direction), a second user input (e.g., a second swipe in a second direction), or a third user input (e.g., a tap on the display device). The mobile device provides a specific manner of displaying a limited set of information (e.g., one or more lead rhythm graphs, which may be selected to launch the array of lead rhythm graphs, or vice versa) to the user on a mobile device, rather than using conventional user interface methods to display a generic list or order of ECG data on a computer or larger mobile device that may require the user to step through multiple menus and/or lead rhythm graphs to find relevant medical information. Display of a single lead rhythm graph on the display device of the mobile device may be advantageous because it avoids a user having to strain to visualize details of a waveform on a relatively small display device. Further, switching between display of a single lead rhythm graph and an array of two or more lead rhythm graphs in response to a tap, and outputting a selected lead rhythm graph from the array may enable a user to avoid scrolling and switching through multiple lead rhythm graphs to select for display a desired lead rhythm graph, which may otherwise be slow, complex, and difficult to learn. Ordering of the set of lead rhythm graphs and simplicity of navigation among the ordered set of lead rhythm graphs may be especially useful in time sensitive situations, such as in the delivery of medical care, especially with respect to displaying limited information due to limited amounts of display area associated with a mobile device.

A technical effect of presenting clinically relevant ECG data on a mobile device in a way that enable sufficient visualization of the waveform and details thereof is the mobility of an ECG diagnostic system (e.g., the mobile device used to view ECG data) may be improved. Additionally, the ordering and selective presentation of relevant lead rhythm graphs (e.g., having relevant medical information, such as waveform abnormalities) improves performance of the mobile device. For example, by presenting only lead rhythm graphs identified as most relevant to a user (e.g., ordering the set of lead rhythm graphs to first present lead rhythm graphs having identified waveform abnormalities and/or lead rhythm graphs which may be conventionally used to supplement diagnosis of an abnormality identified in another lead rhythm graph), a user may not have to scroll through and/or enlarge (e.g., zoom in) multiple lead rhythm graphs to identify one or more lead rhythm graphs of interest. This may improve performance of the mobile device by reducing processing and/or memory demands.

The disclosure also provides support for a method, comprising: receiving electrocardiogram (ECG) data, including a set of lead rhythm graphs, identifying an abnormality in one or more lead rhythm graphs of the set of lead rhythm graphs, ordering the set of lead rhythm graphs based on the abnormality to form an ordered set of lead rhythm graphs, such that a first lead rhythm graph of the ordered set of lead rhythm graphs includes a first waveform which includes the abnormality, and outputting for display a subset of lead rhythm graphs of the ordered set of lead rhythm graphs to a display device of a mobile device, wherein the subset of lead rhythm graphs includes at least the first lead rhythm graph. In a first example of the method, the ECG data further includes a set of analysis results generated by an analysis of the set of lead rhythm graphs, wherein the analysis of the set of lead rhythm graphs includes comparing ECG data of the set of lead rhythm graphs to nominal ECG waveforms, identifying discrepancies among compared ECG data and nominal ECG waveforms as abnormalities, and indicating one or more identified abnormalities in the ECG data of the set of lead rhythm graphs, and outputting the set of analysis results, the set of analysis results including indication of the abnormality. In a second example of the method, optionally including the first example, ordering the set of lead rhythm graphs based on the abnormality further includes positioning a second lead rhythm graph directly after the first lead rhythm graph in the ordered set of lead rhythm graphs, wherein the second lead rhythm graph includes a second waveform that also includes the abnormality included in the first waveform. In a third example of the method, optionally including one or both of the first and second examples further including, in response to receiving a first user input, outputting for display the second lead rhythm graph and not outputting the first lead rhythm graph. In a fourth example of the method, optionally including one or more or each of the first through third examples further including, in response to receiving a first user input, outputting for display a lead rhythm graph of the ordered set of lead rhythm graphs which directly follows the lead rhythm graph in the ordered set of lead rhythm graphs which is being displayed prior to receiving the first user input, and not displaying the lead rhythm graph being displayed prior to receiving the first user input. In a fifth example of the method, optionally including one or more or each of the first through fourth examples, the first user input is a swipe from a left side of the display device to a right side of the display device. In a sixth example of the method, optionally including one or more or each of the first through fifth examples further including, in response to receiving a second user input, outputting for display a lead rhythm graph of the ordered set of lead rhythm graphs which directly precedes the lead rhythm graph in the ordered set of lead rhythm graphs which is being displayed prior to receiving the second user input, and not displaying the lead rhythm graph being displayed prior to receiving the second user input. In a seventh example of the method, optionally including one or more or each of the first through sixth examples further including, in response to receiving a third user input when a single lead rhythm graph of the ordered set of lead rhythm graphs is displayed, outputting for display an array of two or more lead rhythm graphs of the ordered set of lead rhythm graphs, wherein the array displays each lead rhythm graph of the ordered set of lead rhythm graph simultaneously. In an eighth example of the method, optionally including one or more or each of the first through seventh examples, the third user input is a tap on the display device. In a ninth example of the method, optionally including one or more or each of the first through eighth examples further including, in response to receiving the third user input when the array of two or more lead rhythm graphs is displayed, identifying a selected lead rhythm graph of the array of two or more lead rhythm graphs, displaying the selected lead rhythm graph, and not displaying the array of two or more lead rhythm graphs. In a tenth example of the method, optionally including one or more or each of the first through ninth examples, the method further comprises: identifying an orientation of the display device and outputting for display on the display device the subset of lead rhythm graphs, such that a time axis of each lead rhythm graph of the subset of lead rhythm graphs is parallel to a horizontal width of the display device.

The disclosure also provides support for a mobile computing device comprising a display device, the mobile computing device being configured to display on the display device a first lead rhythm graph of a set of twelve lead rhythm graphs of an electrocardiogram (ECG), the first lead rhythm graph launched from an unlaunched state in response to detection of an abnormal rhythm indicator in the first lead rhythm graph by the mobile computing device, the mobile computing device additionally being configured to display on the display device a rhythm summary including a limited list of data from within the set of twelve lead rhythm graphs, each of the data in the limited list being selectable to launch one or more respective lead rhythm graphs from the unlaunched state and enable selected data to be visualized within the one or more respective lead rhythm graphs, and wherein the rhythm summary is displayed while the one or more respective lead rhythm graphs are in the unlaunched state. In a first example of the system, the system further comprises: a user input device, wherein the mobile computing device is configured to receive user input via the user input device and update the rhythm summary in response to the user input. In a second example of the system, optionally including the first example, the display device has a vertical length and a horizontal width, the vertical length being greater than the horizontal width. In a third example of the system, optionally including one or both of the first and second examples, the mobile computing device is further configured to display a time axis of a lead rhythm graph along the horizontal width of the display device when the mobile computing device is in a vertical orientation, such that the horizontal width is parallel with a horizontal axis, and display the time axis of the lead rhythm graph along the vertical length of the display device when the mobile computing device is in a horizontal orientation, such that the vertical length is parallel with the horizontal axis. In a fourth example of the system, optionally including one or more or each of the first through third examples being further configured to order lead rhythm graphs of the set of twelve lead rhythm graphs, including the first lead rhythm graph and lead rhythm graphs which are in the unlaunched state, in an order which is determined in response to detection of the abnormal rhythm indicator. In a fifth example of the system, optionally including one or more or each of the first through fourth examples further configured to, in response to a first user input, transition the first lead rhythm graph of the set of twelve lead rhythm graphs from a launched state to the unlaunched state and transition a second lead rhythm graph from the unlaunched state to the launched state, where the second lead rhythm graph directly follows the first lead rhythm graph in the order of the set of twelve lead rhythm graphs.

The disclosure also provides support for a mobile device comprising: a display device having a vertical length approximately parallel to a direction of gravity, and a horizontal width approximately perpendicular to the direction of gravity, and a computing device operably coupled to the display device and storing instructions executable to: receive a set of lead rhythm graphs illustrating electrocardiogram (ECG) waveform data, identify an abnormality in one or more lead rhythm graphs of the set of lead rhythm graphs, order the set of lead rhythm graphs in an order based on the abnormality in one or more lead rhythm graphs of the set of lead rhythm graphs to form an ordered set of lead rhythm graphs, such that a first lead rhythm graph of the ordered set of lead rhythm graphs includes a first waveform which includes the abnormality, and output for display a subset of the ordered set of lead rhythm graphs based on the order and an orientation of the display device, wherein the subset includes at least the first lead rhythm graph, a time axis of the first lead rhythm graph of the subset of lead rhythm graphs is parallel to the horizontal width, and the first lead rhythm graph is sized to fill dimensions of the display device. In a first example of the system, the computing device is further configured to: output for display to the display device a first section of the first lead rhythm graph when the display device is in a first orientation, wherein in the first orientation the horizontal width is less than the vertical length, and output for display to the display device a second section of the first lead rhythm graph when the display device is in a second orientation, wherein the second section includes the first section, and wherein in the second orientation, the horizontal width is greater than the vertical length. In a second example of the system, optionally including the first example, the display device has a diagonal length of eight inches or less.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method, comprising:
receiving electrocardiogram (ECG) data, including a set of lead rhythm graphs;
identifying an abnormality in one or more lead rhythm graphs of the set of lead rhythm graphs;
ordering the set of lead rhythm graphs based on the abnormality to form an ordered set of lead rhythm graphs, such that a first lead rhythm graph of the ordered set of lead rhythm graphs includes a first waveform which includes the abnormality; and
outputting for display a subset of lead rhythm graphs of the ordered set of lead rhythm graphs to a display device of a mobile device, wherein the subset of lead rhythm graphs includes at least the first lead rhythm graph.

2. The method of claim 1, wherein the ECG data further includes a set of analysis results generated by an analysis of the set of lead rhythm graphs, wherein the analysis of the set of lead rhythm graphs includes comparing ECG data of the set of lead rhythm graphs to nominal ECG waveforms, identifying discrepancies among compared ECG data and nominal ECG waveforms as abnormalities, and indicating one or more identified abnormalities in the ECG data of the set of lead rhythm graphs, and outputting the set of analysis results, the set of analysis results including indication of the abnormality.

3. The method of claim 1, wherein ordering the set of lead rhythm graphs based on the abnormality further includes positioning a second lead rhythm graph directly after the first lead rhythm graph in the ordered set of lead rhythm graphs, wherein the second lead rhythm graph includes a second waveform that also includes the abnormality included in the first waveform.

4. The method of claim 3, further including, in response to receiving a first user input, outputting for display the second lead rhythm graph and not outputting the first lead rhythm graph.

5. The method of claim 1, further including, in response to receiving a first user input, outputting for display a lead rhythm graph of the ordered set of lead rhythm graphs which directly follows the lead rhythm graph in the ordered set of lead rhythm graphs which is being displayed prior to receiving the first user input, and not displaying the lead rhythm graph being displayed prior to receiving the first user input.

6. The method of claim 5, wherein the first user input is a swipe from a left side of the display device to a right side of the display device.

7. The method of claim 5, further including, in response to receiving a second user input, outputting for display a lead rhythm graph of the ordered set of lead rhythm graphs which directly precedes the lead rhythm graph in the ordered set of lead rhythm graphs which is being displayed prior to receiving the second user input, and not displaying the lead rhythm graph being displayed prior to receiving the second user input.

8. The method of claim 5, further including, in response to receiving a third user input when a single lead rhythm graph of the ordered set of lead rhythm graphs is displayed, outputting for display an array of two or more lead rhythm graphs of the ordered set of lead rhythm graphs, wherein the array displays each lead rhythm graph of the ordered set of lead rhythm graph simultaneously.

9. The method of claim 8, wherein the third user input is a tap on the display device.

10. The method of claim 8, further including, in response to receiving the third user input when the array of two or more lead rhythm graphs is displayed, identifying a selected lead rhythm graph of the array of two or more lead rhythm graphs, displaying the selected lead rhythm graph, and not displaying the array of two or more lead rhythm graphs.

11. The method of claim 1, further comprising identifying an orientation of the display device and outputting for display on the display device the subset of lead rhythm graphs, such that a time axis of each lead rhythm graph of the subset of lead rhythm graphs is parallel to a horizontal width of the display device.

12. A mobile computing device (302) comprising a display device (304), the mobile computing device being configured to display on the display device a first lead rhythm graph of a set of twelve lead rhythm graphs of an electrocardiogram (ECG), the first lead rhythm graph launched from an unlaunched state in response to detection of an abnormal rhythm indicator in the first lead rhythm graph by the mobile computing device, the mobile computing device additionally being configured to display on the display device a rhythm summary including a limited list of data from within the set of twelve lead rhythm graphs, each of the data in the limited list being selectable to launch one or more respective lead rhythm graphs from the unlaunched state and enable selected data to be visualized within the one or more respective lead rhythm graphs, and wherein the rhythm summary is displayed while the one or more respective lead rhythm graphs are in the unlaunched state.

13. The mobile computing device of claim 12, wherein the display device has a vertical length (312) and a horizontal width (310), the vertical length being greater than the horizontal width.

14. The mobile computing device of claim 12, being further configured to order lead rhythm graphs of the set of twelve lead rhythm graphs, including the first lead rhythm graph and lead rhythm graphs which are in the unlaunched state, in an order which is determined in response to detection of the abnormal rhythm indicator.

15. The mobile computing device of claim 14, further configured to, in response to a first user input, transition the first lead rhythm graph of the set of twelve lead rhythm graphs from a launched state to the unlaunched state and transition a second lead rhythm graph from the unlaunched state to the launched state, where the second lead rhythm graph directly follows the first lead rhythm graph in the order of the set of twelve lead rhythm graphs.
